# EUROPEAN PATENT APPLICATION

(11) **EP 1 634 566 A1**
(43) Date of publication of application: **15.03.2006**
(21) Application number: 04745723.9
(22) Date of filing: 03.06.2004
(51) Int. Cl.: A61K 8/18, A61K 9/70, A61K 47/34

(54) **COSMETIC ADHESIVE PATCH, METHOD OF USING THE SAME, AND PACKAGE OF COSMETIC ADHESIVE PATCH**

(30) Priority: 03.06.2003 JP 2003158250; 29.09.2003 JP 2003337330; 29.09.2003 JP 2003337331
(71) Applicant: TAKIRON CO., LTD., Osaka-shi, Osaka 541-0052 (JP)
(72) Inventor: SHIKINAMI, Y., Takiron Co., Ltd., Osaka-shi, Osaka 541-0052 (JP); TSUTA, K., Takiron Co., Ltd., Osaka-shi, Osaka 541-0052 (JP); YASUKAWA, K., Takiron Co., Ltd., Osaka-shi, Osaka 541-0052 (JP); OKASAKI, K., Takiron Co., Ltd., Osaka-shi, Osaka 541-0052 (JP); MORRI, H., Takiron Co., Ltd., Osaka-shi, Osaka 541-0052 (JP)
(74) Representative: HOFFMANN EITLE
(86) International application number: PCT/JP2004/008073
(87) International publication number: WO 2004/108097

(57) **Abstract**

The present invention provides a cosmetic patch which can contain therein either one of a water soluble vitamin and a fat soluble vitamin, particularly when the vitamin is vitamin C, can contain and retain vitamin C stably while avoiding its deterioration due to oxidation or hydrolysis and not causing deterioration of the gel, and is equipped with a desired level of adhesive force; a using method of a cosmetic patch capable of effectively transferring vitamin C into the skin from the cosmetic patch; and a cosmetic patch package for storing the cosmetic patch therein.

The present invention relates to a cosmetic patch obtained.by incorporating a vitamin in an adhesive polyurethane gel which has hydrophilic alkylene oxide segments and hydrophobic alkylene oxide segments and has most or all of these segments in liquid form at normal temperature.

## Description

### FIELD OF THE INVENTION

The present invention relates to a cosmetic patch containing a vitamin and using method of the cosmetic patch. The present invention also pertains to a cosmetic patch package having a cosmetic patch containing a water-soluble vitamin, stored therein.

### BACKGROUND ART

Vitamins, for example, vitamin C which is a typical water-soluble vitamin, and vitamin E which is a typical fat-soluble vitamin are known to be effective for skin whitening, pigmentation control, skin anti-aging and skin roughness reduction. As a cosmetic patch having such a vitamin incorporated in a hydrogel serving as a base material, a face pack sheet obtained by incorporating vitamins and the other skin beautifying components in a base material made of an acrylic crosslinked type hydrous gel composed of, as essential ingredients, polyacrylic acid and/or polyacrylate, water and a crosslinking agent is known (for example, refer to Patent Document 1). A face pack having skin beautifying components incorporated in a natural hydrogel such as gelatin or agar is also known.

The above-described face pack sheet using a hydrous gel as a base material, however, has a problem in stability, because a fat-soluble vitamin, which must be added in the emulsified and dispersed form, undergoes separation or transfer with the passage of time owing to lack of compatibility. A water soluble vitamin, on the other hand, can be incorporated in a hydrous gel serving as a base material by making use of its compatibility. Incorporation of water soluble vitamin C (ascorbic acid) which is oxidized and hydrolyzed easily however causes deterioration or coloring of the vitamin by an acidic compound generated by the degradation of a gel. In addition to this problem, the base material made of a hydrous gel undergoes a great change in adhesive force depending on the water content so that a decrease in water content leads to a drastic reduction in adhesive force. [Patent Document 1] Japanese Patent Laid-Open No. 180408/1983

### DISCLOSURE OF THE INVENTION

The present invention is made with a view to overcoming the above-described problems. An object of the present invention is to provide a cosmetic patch capable of containing a vitamin, regardless whether it is water soluble or fat soluble, particularly, a cosmetic patch which can stably contain and retain Vitamin C without causing deterioration of the gel which will otherwise occur by its oxidation and hydrolysis, and has a desired level of adhesive force. Another object of the present invention is to provide a using method of a cosmetic patch capable of effectively transferring, from the cosmetic patch to the skin, Vitamin C stably incorporated and retained in the patch and making the skin absorb it.

A further object of the present invention is to provide a cosmetic patch package equipped with a desired level of adhesive force, which package can avoid deterioration of a water soluble vitamin (particularly, vitamin C) due to its oxidation and hydrolysis, can be stored stably without causing deterioration of a gel which will otherwise occur by an acidic substance generated by the oxidation and hydrolysis of the vitamin, and can effectively release the water soluble vitamin when applied.

The present inventors have carried out an intensive investigation in order to overcome the above-described problems. As a result, it has been found that by incorporating a vitamin in a specific gel, the gel can stably contain the vitamin while avoiding deterioration of it due to its oxidation and hydrolysis and also avoiding deterioration of the gel and have a desired level of adhesive force, leading to the completion of the invention.

A cosmetic patch according to the present invention is characterized in that:
(1) it comprises an adhesive polyurethane gel comprising hydrophilic alkylene oxide segments and hydrophobic alkylene oxide segments and having most or all of these segments in liquid form at normal temperature, and a vitamin contained therein,
(2) in the above (1), the hydrophilic alkylene oxide segments are ethylene oxide chains and the hydrophobic alkylene oxide segments are propylene oxide chains,
(3) in the above (2), the polyurethane gel has ethylene oxide chains and propylene oxide chains at a molar ratio of from 50 to 90 : from 50 to 10 and the vitamin is a water soluble vitamin,
(4) in the above (2), the polyurethane gel has ethylene oxide chains and propylene oxide chains at a molar ratio of from 50 to 10 : from 50 to 90 and the vitamin is a fat soluble vitamin,
(5) in the above (1), the vitamin is incorporated in an adhesive polyurethane gel comprising segments made of a copolymer of ethylene oxide and propylene oxide and having most or all of these segments in liquid form at normal temperature,
(6) in the above (1) or (5), the polyurethane gel forms a gel layer,
(7) in any one of the above (1), (3), (5) and (6), the vitamin is vitamin C and the polyurethane gel is free of a solvent for vitamin C, or
(8) in the above (6), the gel layer has, on one surface thereof, a nonwoven fabric and a resin film stacked one after another.
   The using method of a cosmetic patch according to the present invention is characterized in that:
(9) just before use of a cosmetic patch obtained by incorporating a water soluble vitamin in a gel layer, which is made of an adhesive polyurethane gel comprising ethylene oxide segments and propylene oxide segments, or segments composed of a copolymer of ethylene oxide and propylene oxide and having most or all of these segments in liquid form at normal temperature, in a dry state in the absence of a solvent for the water soluble vitamin, impregnating the gel layer with water to dissolve the water soluble vitamin therein; and applying the cosmetic patch to the skin to transfer the water soluble vitamin and water into the skin; or
(10) in the above (9), water is added to the cosmetic patch, which has been stored in a concave body while having a net made of a synthetic resin laid over the bottom surface of the patch, to impregnate the patch with water and after removal of the net, the cosmetic patch is applied to the skin.
   The cosmetic patch package according to the present invention is characterized in that:
(11) it comprises a cosmetic patch which comprises a substantially water-free adhesive polyurethane gel which has hydrophilic alkylene oxide segments and hydrophobic alkylene oxide segments and has most or all of these segments in liquid form at normal temperature, and a water soluble vitamin contained therein;
   a tray having formed therein a storing recess for storing the cosmetic patch, and
   a cover unit which can be laid over the tray, has a water reservoir containing water to be fed to the storing recess, and opens the bottom surface of the reservoir by the application of an external force to the reservoir;
(12) it comprises a cosmetic patch which comprises a substantially water-free adhesive polyurethane gel which has hydrophilic alkylene oxide segments and hydrophobic alkylene oxide segments and has most or all of these segments in liquid form at normal temperature, and a water soluble vitamin contained therein;
   a tray having formed therein a storing recess for storing the cosmetic patch, and
   a water reservoir bag which is stored in the storing recess of the tray after laid over the cosmetic patch, and opens by the application of an external force;
(13) in the above (11) or (12), the water soluble vitamin is vitamin C,
(14) in the above (11) or (12), the polyurethane gel has hydrophilic ethylene oxide segments and hydrophobic propylene oxide segments, or have segments composed of a copolymer of hydrophilic ethylene oxide and hydrophobic propylene oxide,
(15) in the above (11), the water reservoir of the cover unit laid over the tray is located above the storing recess,
(16) in the above (11), the storing recess is formed at two sites of the tray, a connecting channel for connecting these two storing recesses is formed in the tray, and the cover unit is laid over the tray so that the water reservoir is located above the connecting channel,
(17) in any one of the above (11) to (16), the storing recess of the tray has small irregularities formed on the bottom surface thereof;
(18) in any one of the above (11) to (16), a net is laid over the bottom surface of the storing recess of the tray;
(19) in the above (11), an opening leading to the exterior is formed between the tray and the cover unit to be laid thereover;
(20) in the above (16), one of the tray and cover is equipped with a protrusion and the other one is equipped with a recess in which the protrusion is fit so that when they are fitted, the water reservoir is located above the connecting channel of the tray; or
(21) in the above (11), the tray and cover unit are formed so that the periphery of one of the tray and cover protrudes from that of the other one when they are overlapped each other.

The term "substantially water-free" as used herein means that a minute amount of water is contained, and that water is contained in an amount as small as 1 wt% or less, preferably 0.5 wt% or less, more preferably 0.3 wt% or less.

In the cosmetic patch of the invention, when a vitamin contained therein is water soluble, it exhibits an affinity for the hydrophilic alkylene oxide segments of the polyurethane gel which are in liquid form at normal temperature and is contained and retained in these segments, while when it is fat soluble, it exhibits an affinity for the hydrophobic alkylene oxide segments of the polyurethane gel which are in liquid form at normal temperature and is contained and retained in these segments. Such a vitamin solution or a vitamin in liquid form is retained among the three-dimensional network chains of the polyurethane gel. As described above, the cosmetic patch in a first aspect of the invention can contain and retain a vitamin more effectively and release it more smoothly compared with the conventional one, because the vitamin, regardless whether it is water soluble or fat soluble, exhibits a strong affinity for and is retained well in either one of the hydrophilic and hydrophobic segments of the polyurethane gel. Moreover, the polyurethane gel has a good adhesive force and always maintains a stable adhesive force by selecting a proper molecular form of the alkylene oxide segments in liquid form at normal temperature.

The cosmetic patch containing a water soluble vitamin such as vitamin C shows a good release rate of it and a good adhesive force when the polyurethane gel has ethylene oxide chains (EO chains) and polypropylene chains (PO chains) at a molar ratio falling within a range of from 50 to 90 : from 50 to 10. When the molar ratio of the EO chains is below the above-described range, the hydrophobicity of the polyurethane gel increases, leading to a reduction in the transfer and release efficiency, to the skin, of the water soluble vitamin dissolved in water as described later in detail. When the molar ratio of the EO chains exceeds the above-described range, on the other hand, total deterioration in the adhesive force and excessive increase in the affinity for water occur, leading to a reduction in the total release rate. At a molar ratio of the PO chains below the above-described range, the polyurethane gel has a decreased affinity with a fat soluble vitamin owing to a stronger hydrophilicity, while at a molar ratio of the PO chains exceeding the above-described range, a release rate lowers owing to the excessive affinity. Thus, molar ratios outside the above-described range lead to inconveniences.

In the case where a fat soluble vitamin is added, an affinity with the fat soluble vitamin and release rate are desirable when the polyurethane gel has EO chains and PO chains at molar ratios falling within a range of from 50 to 10 : from 50 to 90. Molar ratios of the PO chains below the above-described range heighten the hydrophilicity of the polyurethane gel and lower the affinity with the fat soluble vitamin, while molar ratios of the PO chains exceeding the above-described range lower the release rate owing to excessive affinity. Thus, molar ratios outside the above-described range lead to inconveniences.

In the cosmetic patch of the invention, when the vitamin is water soluble, it has an affinity for the EO portion of the segments and is contained and retained therein while partially showing a behavior near dissolution. When the vitamin is fat soluble, on the other hand, it has an affinity for the PO portion of the segments and is contained and retained therein while partially showing a behavior near dissolution. Accordingly, this cosmetic patch can also contain both a water soluble vitamin and a fat soluble vitamin. By adjusting a molar ratio of EO to PO in a similar manner to that described above, the amount of a vitamin which can be retained with a good affinity can be increased.

In these cosmetic patches of the present invention, when a nonwoven fabric and a resin film are stacked over one of the surfaces of the gel layer, strength of them can be heightened by the reinforcing effect of the nonwoven fabric. In addition, since one of the adhesive surfaces of the gel layer is covered by the resin film, the resulting cosmetic patches become easy to handle without making fingers sticky. Moreover, a release efficiency increases because a vitamin is released only from the other surface of the gel layer.

When vitamin C is incorporated in the polyurethane gel of these cosmetic patches, a time-dependent deterioration of vitamin C due to the oxidation and hydrolysis of it can be prevented by subjecting the gel to a state not containing a solvent (mainly, water) for Vitamin C. This is because vitamin C out of contact with the solvent can be stored stably in the gel.

According to the using method of the cosmetic patch of the invention, since a water soluble vitamin is contained and stored in the gel layer in a dry state in the absence of a solvent until just before use, no deterioration of a water soluble vitamin due to oxidation and hydrolysis occurs and there is therefore no fear of the gel being deteriorated by an acidic substance which will otherwise be generated by this deterioration. Just before use, the gel layer of the cosmetic patch is impregnated with water which is a good solvent to dissolve the water soluble vitamin in water so that the water soluble vitamin and water are transferred and absorbed into the skin as soon as the cosmetic patch is applied to the skin. The cosmetic patch containing vitamin C as the vitamin shows effects such as whitening, pigmentation reduction and antioxidant ones.

In the using method according to the present invention, when water is added to the cosmetic patch, which has been stored in a concave body while having a net made of a synthetic resin laid over the bottom surface of the cosmetic patch, to cause the patch to absorb water and then, the resulting cosmetic patch is applied to the skin after removal of the net, the water soluble vitamin contained in the gel layer is dissolved therein uniformly and can be transferred and absorbed into the skin, because water thus added runs toward the bottom surface side of the cosmetic patch along the net and is absorbed in the gel layer of the cosmetic patch uniformly from the whole bottom surface of the cosmetic patch.

When vitamin C (L-ascorbic acid), which is a typical example of water soluble vitamins, is contained in the cosmetic patch, a trade-off as described below exists.

As shown by the below-described reaction scheme, vitamin C [pure L-ascorbic acid (1)] is easily oxidized into dehydroascorbic acid (2), followed by hydrolysis. Since it is a substance having such a chemical structure, it is considerably unstable even under normal state circumstances at room temperature. In particular, it is markedly unstable when dissolved in water. It has been confirmed that eight hours after it is dissolved in water, at least about 10% of it has already been oxidized into dehydroascorbic acid (2) and deactivated as a result of hydrolysis into a substance (3), (4), (5), (6) or (7) having a corresponding chemical structural formula.

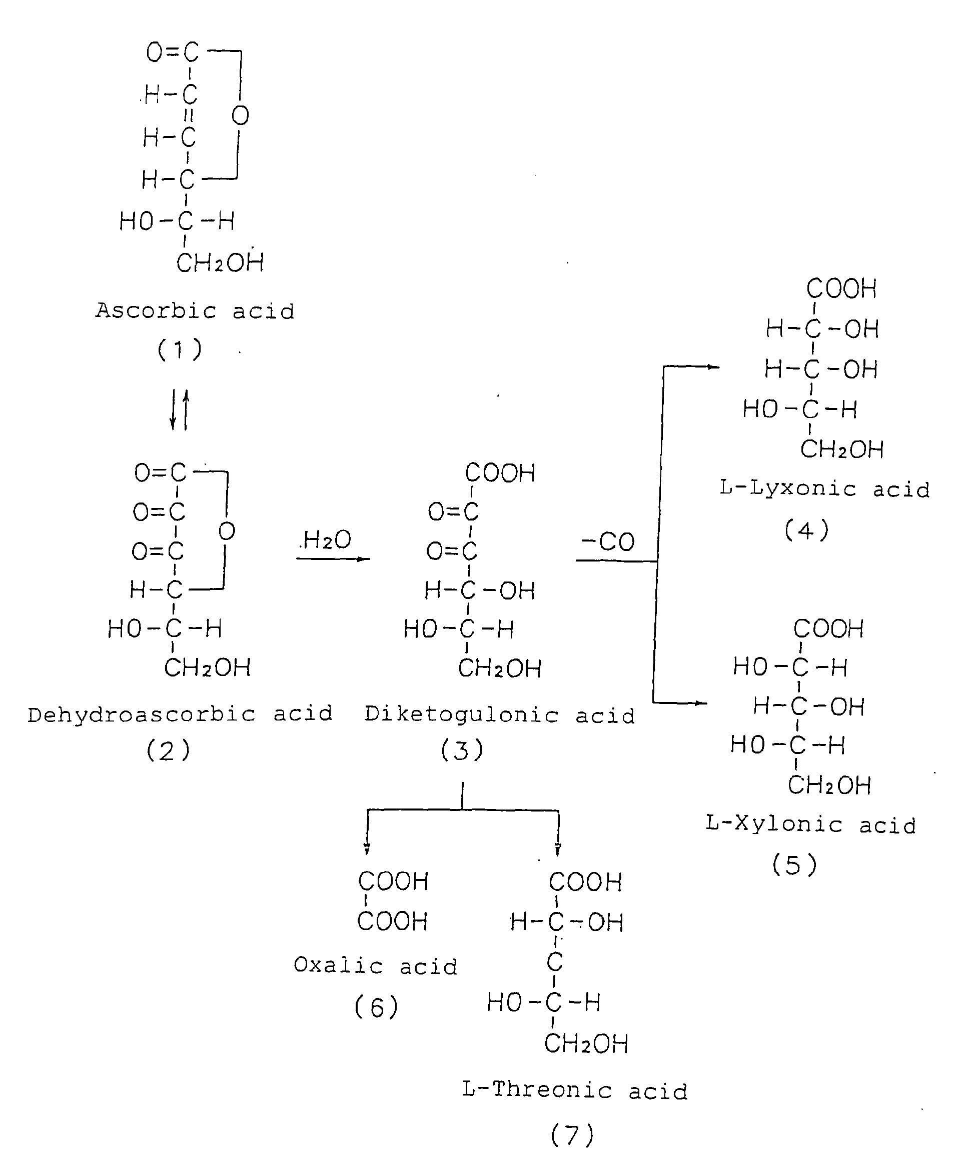

Vitamin C [pure L-ascorbic acid (1)] is a crystalline solid in white powder form having a melting point of 190 to 192°C. Only water is a good solvent for it. It is sparingly soluble in polar solvents such as alcohol (1 g of it dissolves in 3 ml of water, 30 ml of an alcohol, 50 ml of an absolute alcohol, 100 ml of glycerol or 20 ml of propylene glycol, while it is insoluble in chloroform, benzene or ether).

Accordingly, when L-ascorbic acid (1) [or a mixture with dehydroascorbic acid (2)] is caused to exist with water in the reservoir layer of a cosmetic patch, L-ascorbic acid (1) easily degrades, turns yellow and then turns brown with the passage of time during the storage of the product.

When solubility is taken into consideration, water is a good solvent for L-ascorbic acid (1) (a large amount of an alcohol is required to dissolve it therein) and is indispensable for transfer and release of L-ascorbic acid (1), together with water as a solvent therefor, to the skin after application of a cosmetic patch to the skin. There exists a trade-off, that is, water is necessary for the effective release of L-ascorbic acid (1), though it is easily oxidized in water and then hydrolyzed by water.

For the development of such a cosmetic patch, various hydrogels equipped with both functions as a reservoir layer of L-ascorbic acid (1) and an adhesive layer to the skin have so far been investigated. L-ascorbic acid (1) easily degrades and its discoloration and quality change are inevitable, while hydrogels are always deteriorated and even lose their shape by the time-dependent quality change due to an acidic substance produced by the degradation of L-ascorbic acid (1). The present invention provides a means for solving this trade-off.

The adhesive polyurethane gel of the present invention functioning both as a storing layer of vitamin C and an adhesive layer to the skin is a non-hydrogel and it is principally a water-free. It is an amphiphilic substance composed of a mixture of hydrophilic ethylene oxide (EO) chains and hydrophobic propylene oxide (PO) chains and its segments are in liquid form at normal temperature. It is a so-called one-component type special adhesive gel which is in gel form even without containing water.

The gel can be made either hydrophilic or hydrophobic by changing a molar ratio of EO chains to PO chains. In the case of a cosmetic patch containing water soluble vitamin C, segments having an increased molar ratio of hydrophilic EO chains may be selected. By adding water such as lotion to the gel containing vitamin C from the surface of the gel and swelling it with water, vitamin C can be dissolved in the resulting gel. The cosmetic patch is then applied to the face skin, whereby vitamin C can be transferred into the skin, lead by the body fluid in the skin. During storage before application of the patch, vitamin C can be stored in almost water-free dry state so that time-dependent degradation of vitamin C can be suppressed sufficiently until just before use.

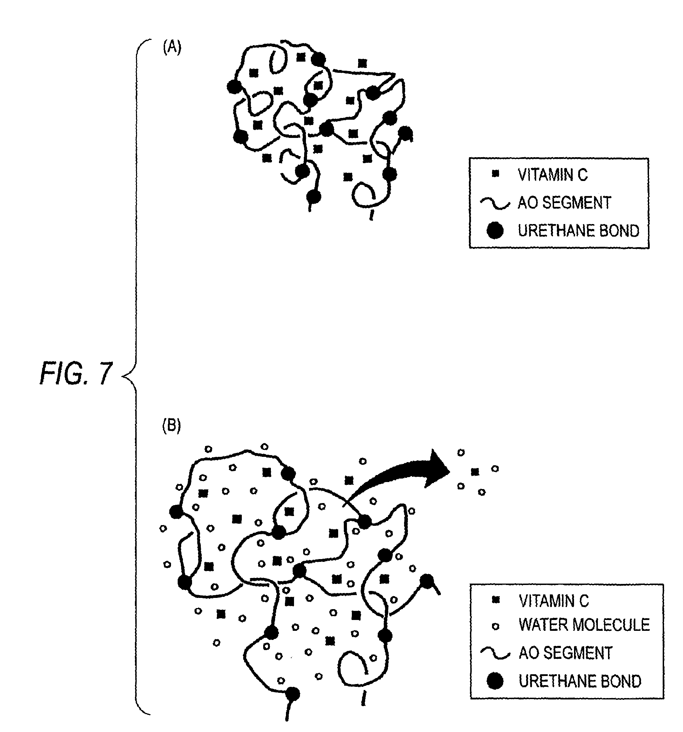

Fig. 7 is a schematic view illustrating vitamin C contained in a dry polyurethane gel and that contained in the gel swelled with water. As illustrated in FIG. 7(A), in an almost water-free dry state, vitamin C is dispersed among the alkylene oxide (AO) segments of the polyurethane segments and this state is maintained over a prolonged period without transfer or degradation of vitamin C. When water is added, on the other hand, water molecules enter among the AO segments as illustrated in Fig. 7(B) and the gel is swelled with them and at the same time, vitamin C dissolves in the water. When the gel under such a state is applied to the human face, vitamin C and water transfer into the face skin.

Accordingly, the cosmetic patch and using method thereof satisfying the two trade-off conditions can thus be completed.

With regards to a cosmetic patch containing vitamin E which is fat soluble, vitamin E is almost insoluble in water and readily soluble in an alcohol (95% in ethanol) so that it is incorporated (about 5 to 10 wt%) in the polyol of a polyurethane gel having predetermined segment chains in a good affinity form (as a solution or dispersion), or it is added dropwise to the gel after dissolved in ethanol. Since it is impossible to transfer Vitamin E in the gel and release it to the skin after its application, the purpose of the application is attained by adding a liquid dispersant or humectant (this also enables effective transfer and penetration of it into the skin and a humectant such as polyethylene glycol or glycerin or liquid surfactant is used, for example). At this time, segments having a proper PO:EO molar ratio may be selected after due consideration of the affinity.

A mixture of vitamin B and vitamin E means a mixture of a water soluble vitamin and a fat soluble vitamin. One of preferable methods is to select a polyurethane gel having segments composed of a random copolymer having a EO : PO molar ratio of 50:50 and add the mixture to such a gel. Vitamin B is relatively stable, while vitamin E is also relatively stable, though it is oxidized and becomes dark red by exposure to air or light. A severe prevention measure against degradation as required for vitamin C is therefore not necessary.

The cosmetic patch package of the present invention comprises at least a cosmetic patch and a tray for storing it and the polyurethane gel of the cosmetic patch is a substantially water-free gel. Since a water soluble vitamin such as vitamin C can be contained stably in the gel in a dry state, hydrolysis of the vitamin does not occur and neither quality change nor deterioration of the vitamin therefore occurs even after passage of time. The polyurethane gel is also free from deterioration which will otherwise occur by an acidic substance generated by the degradation. In addition, this polyurethane gel is an anhydrous gel having most or all of the segments in liquid form at normal temperature so that it exhibits a good and stable adhesive force different from the conventional hydrogels whose adhesive force undergoes a great change, depending on a change in the water content.

In the cosmetic patch package having a tray to be overlaid with a cover unit, when the bottom surface of the cover unit is opened for use by the application of an external force to a water reservoir of the cover unit, water flows from the water reservoir into a storing recess, penetrates and is absorbed into the polyurethane gel of the cosmetic patch, and a water soluble vitamin dissolves in water thus absorbed. By applying the cosmetic patch to the face after removal of the cover unit from the cosmetic patch package, the water soluble vitamin dissolved in water transfers from the polyurethane gel to the skin and is absorbed smoothly thereinto. The cosmetic patch, when it contains vitamin C, can exhibit effects such as whitening, pigmentation reduction and antioxidant ones.

In the cosmetic patch package having a cosmetic patch stored in a storing recess of a tray with a water reservoir bag being laid over the cosmetic patch, when an external force is applied to the water reservoir bag to open it at the time of use, water penetrates and is absorbed into the polyurethane gel of the cosmetic patch and a water soluble vitamin such as vitamin C dissolves in water. By applying this cosmetic patch to the face to cause transfer and absorption of the water soluble vitamin to the face skin, it can also exhibit the above-described effects.

When the polyurethane gel has hydrophilic ethylene oxide segments and hydrophobic propylene oxide segments, or has segments composed of a copolymer of hydrophilic ethylene oxide and hydrophobic propylene oxide, vitamin C has an affinity for the hydrophilic ethylene oxide segments or ethylene oxide portion of the copolymer segments, and it is contained and retained in the polyurethane gel while partially exhibiting a behavior like dissolution. In addition, its transfer to the skin is smooth. But, a polyurethane gel composed only of hydrophilic ethylene oxide segments is not desired because an amount of vitamin C transferred to the skin decreases owing to its too strong force to retain vitamin C.

The cosmetic patch package having the water reservoir of the cover unit placed above the storing recess of the tray has an advantage that water flows into the storing recess without leakage to the outside when the bottom surface of the water reservoir is opened. The cosmetic patch package having a cover unit laid over the tray so that a water reservoir is located above a connecting channel for connecting two storing recesses formed in the tray has, on the other hand, an advantage that only one opening operation of the water reservoir is necessary because when the bottom surface of the water reservoir is opened by applying an external force thereto, water flows uniformly from the water reservoir into two storing recesses through the connecting channel. The cosmetic patch package having small irregularities formed on the bottom surface of the storing recess of the tray or having a net laid over the storing recess of the tray has an advantage that water fed to the storing recess runs toward the reverse side of the cosmetic patch along the irregularities or net on the bottom surface, and is absorbed uniformly into the polyurethane gel of the cosmetic patch.

In the cosmetic patch package having an opening formed between the tray and the cover of the tray leading to the outside, oxidation and hydrolysis of vitamin C and deterioration of the polyurethane gel can be suppressed further by putting the package and either one or both of a desiccant and an oxygen absorber in an outer bag having a gas barrier property, thereby causing its(their) effects on the inside of the package to remove either one or both of water and oxygen. In the package having a tray and a cover unit equipped with a protrusion and a recess, respectively, the whole amount of water in the water reservoir can be fed to the storing recess through a connecting channel and is impregnated in the polyurethane gel, because the water reservoir of the cover unit can be located above the connecting channel of the tray precisely by overlapping the protrusion and the recess to fit the former in the latter. The package has an advantage that in the cosmetic patch package having a structure permitting protrusion of the periphery of either one of the tray and cover unit from that of the other one when they are overlapped each other, the cover unit can be peeled from the tray easily by holding the protruded portion in hand.

### BROEF DESCRIPTION OF THE DRAWINGS

Fig. 1 is a cross-sectional view of a cosmetic patch according to one embodiment of the present invention.
Fig. 2 is a plan view illustrating one example of a package having the cosmetic patch stored therein while partially cutting away its surface cover film.
Fig. 3 is a cross-sectional view taken along a line A-A of Fig. 2.
Fig. 4 is a plan view illustrating another example of a package having various shapes of cosmetic patches of the present invention stored therein in combination while omitting its surface cover film.
Fig. 5 is a cross-sectional view explaining one embodiment of the using method of a cosmetic patch according to the present invention.
Fig. 6 is a cross-sectional view illustrating dropwise addition and absorption of an aqueous solution of vitamin C in a polyurethane gel over a net which made of a synthetic resin has been laid.
Fig. 7(A) is a schematic view illustrating Vitamin C contained in a dry polyurethane gel, while Fig. 7(B) is a schematic view illustrating this polyurethane gel swelled with water.
Fig. 8 is a partial cutaway plan view of a cosmetic patch package according to one embodiment of the present invention.
Fig. 9 is a cross-sectional view taken along a line A-A of Fig. 8.
Fig. 10 is an exploded cross-sectional view of the cosmetic patch package.
Fig. 11 is an explanatory view of a using method of the cosmetic patch package.
Fig. 12 is an explanatory view illustrating one example of a manufacturing method of the cosmetic patch package.
Fig. 13 is a partial cutaway plan view of a cosmetic patch package according to another embodiment of the present invention.
Fig. 14 is a cross-sectional view taken along a line B-B of Fig. 13.
Fig. 15 is a cross-sectional view taken along a line C-C of Fig. 13.
Fig. 16 is a cross-sectional view illustrating a cosmetic patch package according to the one embodiment of the present invention hermetically sealed in an outer bag together with an oxygen absorber and a desiccant.
Fig. 17(a) is a plan view illustrating a tray of a cosmetic patch package according to a further embodiment of the present invention, while Fig. 17(b) is a plan view of a cover unit to be overlapped with the tray of Fig. 17(a).
Fig. 18 is a cross-sectional view taken along a line D-D of Fig. 17(a).
Fig. 19 is a cross-sectional view taken along a line E-E of Fig. 17(a).
Fig. 20 is a cross-sectional view taken along a line F-F of Fig. 17(b).
Fig. 21 is a front center longitudinal cross-sectional view illustrating the cosmetic patch package according to the one embodiment of the present invention hermetically sealed in an outer bag together with a desiccant and an oxygen absorber.
Fig. 22 is a partial cutaway plan view of a cosmetic patch package according to a still further embodiment of the present invention.
Fig. 23 is a cross-sectional view illustrating the cosmetic patch package according to the still further embodiment of the present invention.
Fig. 24 is an exploded cross-sectional view illustrating the cosmetic patch package according to the still further embodiment of the present invention.
Fig. 25 is a cross-sectional view illustrating a cosmetic patch package according to a still further embodiment of the present invention.
Fig. 26 is a partially enlarged cross-sectional view illustrating a water reservoir bag of the cosmetic patch package.

In the drawings, indicated by the numeral 1 is a gel layer of a polyurethane gel, 2 a nonwoven fabric, 3 a resin film, 4 a tray, 5 a recess of the tray, 7 a net made of a synthetic resin, 10, 11, 12, 13 and 14 each a cosmetic patch, P₁, P₂, P₃, P₄, P₅, P₆, and P₇ each a cosmetic patch package, 101 a tray, 101a a storing recess, 101b a connecting channel, 101c small irregularities, 102 a net, 103 a cosmetic patch having a water soluble vitamin incorporated in its polyurethane gel, 104 a cover film, 105 a cover unit, 105a a water reservoir, 105b a protrusion, 105c and 105d each a cover film, 105e an opening leading to the outside, 101f a protrusion, 105g an oxygen absorber, 105h a desiccant, 105i an outer bag, 106 water, 109 a water reservoir bag, and 109a a notch.

### BEST MODE FOR CARRYING OUT THE INVENTION

Specific embodiments of the present invention will next be described in detail with reference to accompanying drawings. It should however be borne in mind that the present invention is not limited to or by them.

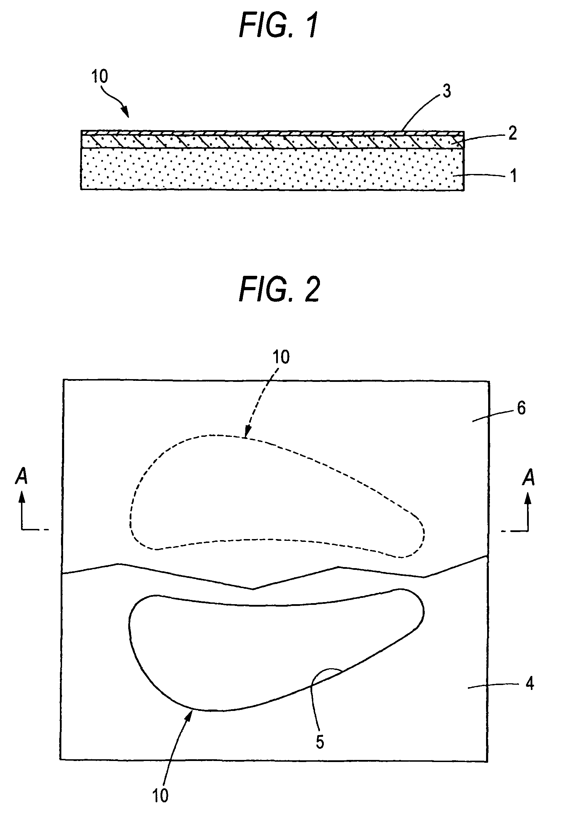

Fig. 1 is a cross-sectional view of a cosmetic patch according to one embodiment of the present invention.

This cosmetic patch 10 has a nonwoven fabric 2 and a resin film 3 stacked one after another over the upper surface of a gel layer 1 composed of an adhesive polyurethane gel. The nonwoven fabric 2 is embedded in the surface layer of the gel layer 1 and the gel layer 1 has a vitamin contained and retained therein.

The polyurethane gel constituting the gel layer 1 has hydrophilic alkylene oxide segments and hydrophobic alkylene oxide segments and it is an adhesive polyurethane gel having most or all of these segments in liquid form at normal temperature. This polyurethane gel is a so-called interpenetrated network segment polyurethane gel available by using one or more of polyol components A to D and one or more of polyisocyanate components E to I, which are represented by the below-described structural formulas, and forming a urethane bond between the -OH group and -NCO group of these components, respectively.

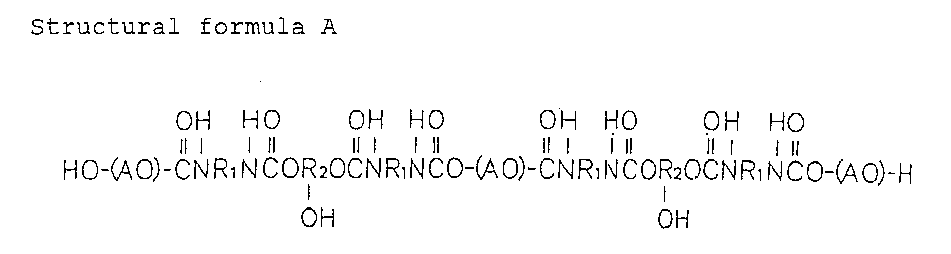

R₁ and R₂ each represents any one of alkyl compounds, alicyclic compounds and aromatic compounds and AO means an alkylene oxide segment.

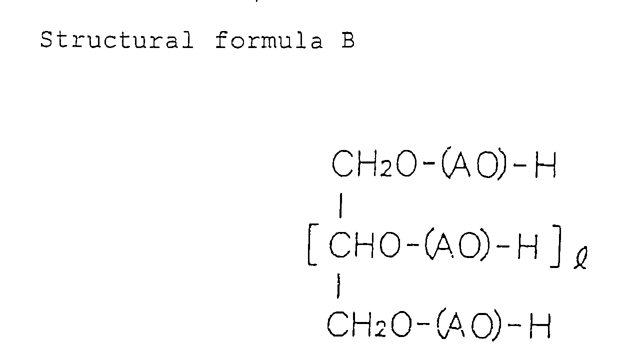

AO means an alkylene oxide segment and 1 stands for an integer of 1 or 4.

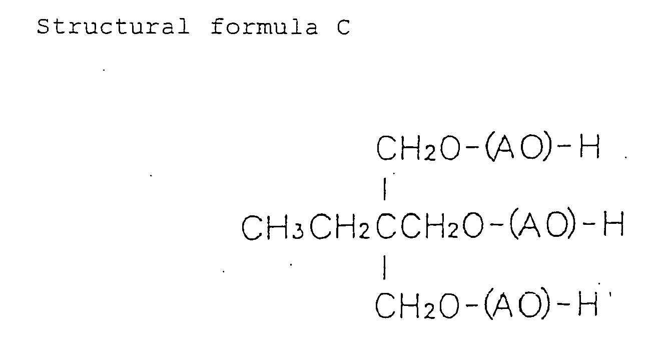

AO means an alkylene oxide segment.

### Structural formula D

RO-(AO)-H

AO means an alkylene oxide segment, and R represents any one of hydrogen atom, alkyl compounds, alicyclic compounds, and aromatic compounds.

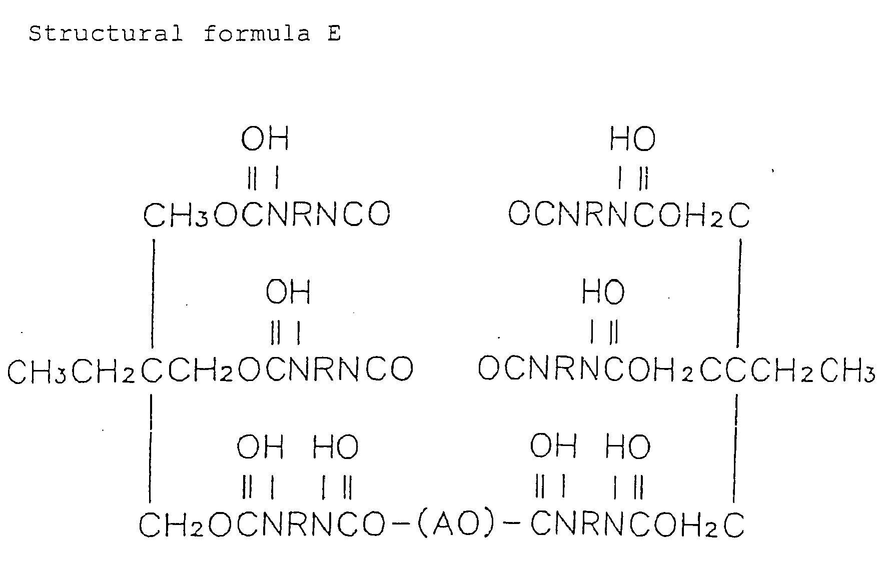

R represents any one of allcyl groups, alicyclic compounds, and aromatic compounds and AO means an alkylene oxide segment.

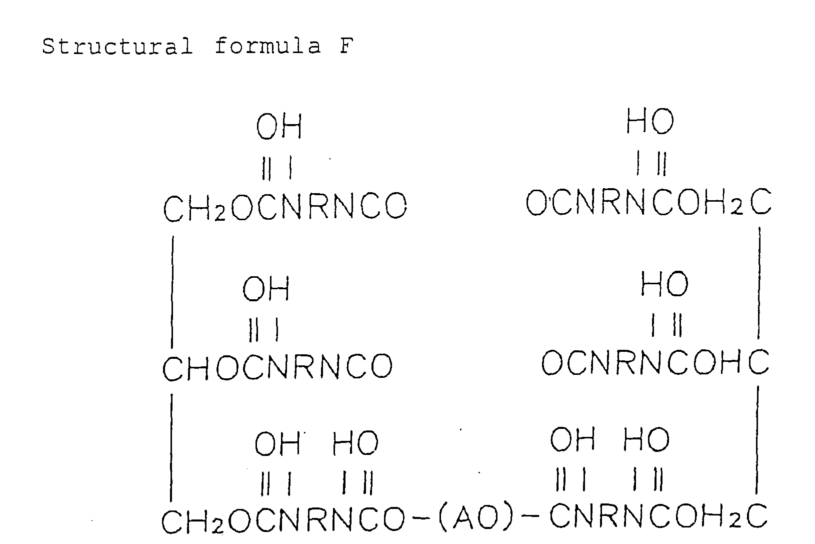

R represents any one of alkyl groups, alicyclic compounds, and aromatic compounds and AO means an alkylene oxide segment.

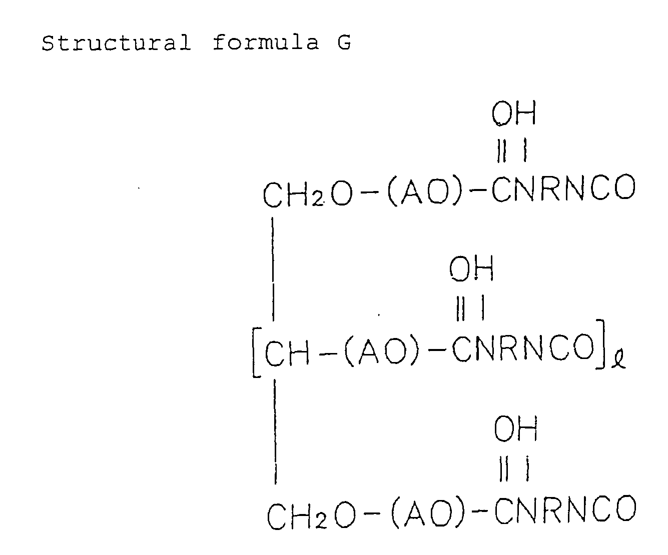

R represents any one of alkyl groups, alicyclic compounds, and aromatic compounds, AO means an alkylene oxide segment and 1 stands for an integer of 1 or 4.

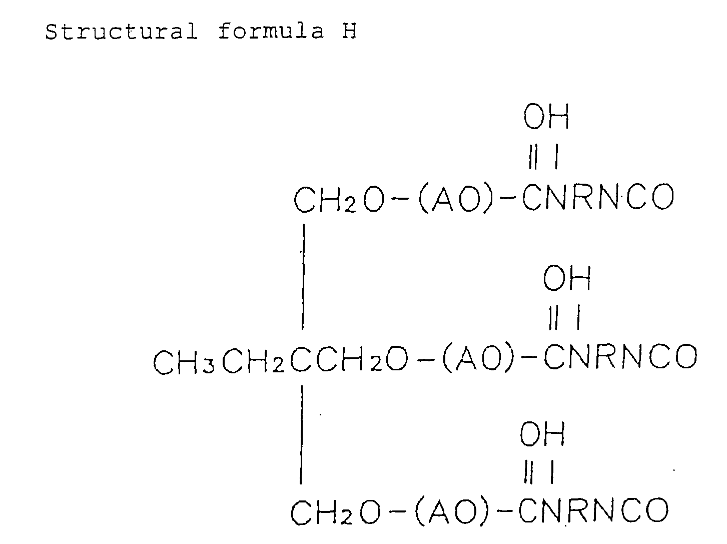

R represents any one of alkyl groups, alicyclic compounds, and aromatic compounds, and AO means an alkylene oxide segment.

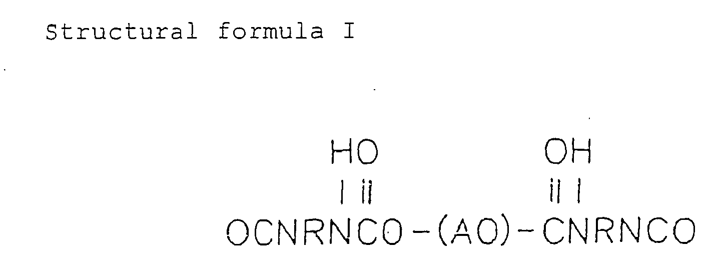

R represents any one of alkyl groups, alicyclic compounds, and aromatic compounds, and AO means an alkylene oxide segment.

Prepolymers as the polyol components represented by the structural formulas A to D will next be described. The compound represented by the structural formula A is a polyurethane polyol prepolymer which is a reaction product between a polyether polyol and a diisocyanate and it has, as components at both ends, a polyether polyol and has an -OH group as an end group.

The diisocyanate compound used here is equal to that in the prepolymer of polyurethane polyisocyanate which will be described later and phenylene diisocyanate, 2,4-toluylene diisocyanate (TDI), 4,4'-diphenylmethane diisocyanate (MDI), naphthalene-1,5-diisocyanate, hexamethylene diisocyanate (HMDI), tetramethylene diisocyanate (TMDI), lysine diisocyanate, xylylene diisocyanate (XDI), hydrogenated TDI, hydrogenated MDI, dicyclohexyldimethylmethane p,p'-diisocyanate, diethyl fumarate diisocyanate and isophorone diisocyanate (IPDI) can be exemplefied.

The compound represented by the structural formula B is glycerol (1=1) or sorbitol (1=4) added with a polyether polyol, while that represented by the structural formula C is trimethylol propane added with a polyether. An adduct, with a polyether polyol, of a polyalcohol such as 1,2,6-hexanetriol of the below-described structural formula J, trimethylolethane of the structural formula K, pentaerythritol of the structural formula L or polyglycerin of the structural formula M (n = an integer of from 2 to 30) or a partial ester thereof can also be used.

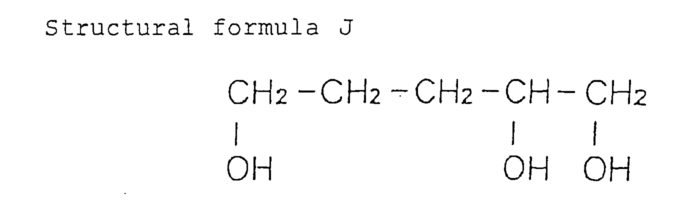

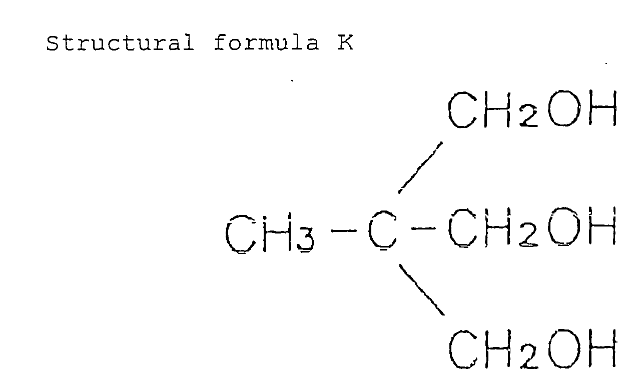

### Structural formula L

C(CH₂OH)₄

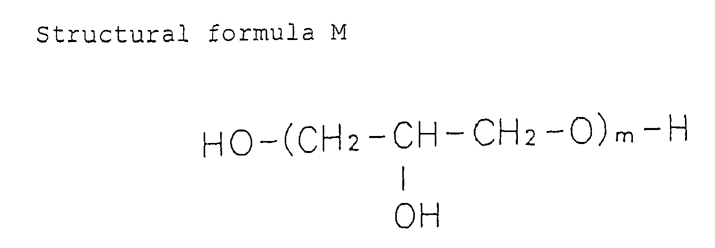

The compound represented by the structural formula D is a polyether polyol having an alkylene oxide segment. It two types, one has an -OH group at both ends thereof and the other one has one end blocked with an alkyl group or an aromatic group. It is easily available as a commercial product.

The polyisocyanate prepolymers represented by the structural formulas E to I will next be described. The prepolymer represented by the structural formula E is obtained by dimerizing two molecules of a triisocyanate, which has been obtained by reacting trimethylolpropane with a diisocyanate, with one molecule of AO and it is a tetraisocyanate, a tetrafunctional compound. The compound represented by the structural formula F is available in a similar manner except for the use of glycerol instead of trimethylolpropane. Preparation of such a tetraisocyanate needs delicate adjustment of the reaction, because dimerization of a triisocyanate with 2 or 3 molecules of AO tends to occur. A portion of the triisocyanate therefore remains unreacted in the resulting tetraisocyanate. Existence of unreacted triisocyanate however causes a difference in the molecular size of segment polyurethane upon reaction with a polyol and is sometimes useful for the control of adhesion properties.

The compound of the structural formula G is obtained by reacting the polyol of the structural formula B with a diisocyanate, while the compound represented by the structural formula H is obtained similarly by reacting the polyol of the structural formula C with a diisocyanate and it is trifunctional. The compound of the structural formula I is a reaction product of a polyether polyol with a diisocyanate and it is bifunctional.

The alkylene oxide segment represented by AO in the above-described structural formula A to I is, to be more specific, any one of a hydrophilic EO chain, a hydrophobic PO chain or an EO-PO copolymer [for example, a block copolymer represented by the below-described structural formula N, an alternating copolymer such as -(PO-EO-EO-PO)ₘ-, -(PO-PO-EO)ₘ-, -(EO-EO-PO)ₘ- or - (EO-PO-EO-PO)ₘ- (wherein, m stands for an integer of 1 or greater) or a random copolymer of EO and PO].

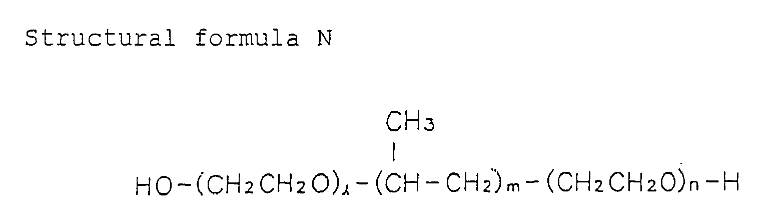

In order to obtain a polyurethane gel having excellent adhesion properties at normal temperature and having a good affinity for vitamins, most or all of the AO segments each composed of such an EO chain, PO chain or EO-PO chain must be in liquid form at normal temperature. For this purpose, their molecular weight is controlled. The EO chain has adequately an MW of from 150 to 1000, preferably from 300 to 800. The PO chain is in liquid form when it has a molecular weight of tens of thousands so that it can be used within a wide range of a molecular weight. The smaller the ratio of an end group, however, the lower the reaction probability. In addition, when it is a too long segment, the resulting polyurethane gel becomes rich in fluidity and poor in shape retention so that a molecular weight preferably falls within a range of about 200 to several thousand.

The EO-PO copolymer differs in fluidity (softness), adhesion properties and affinity for vitamins, depending on its component ratio or arrangement. When the molar ratio of PO of the block copolymer is high, it is in liquid form even if it has a high molecular weight. Even if the molar ratio of PO is low, the block copolymer is in liquid form when the EO has a low molecular weight. As described above, however, a higher molecular weight leads to problems such as poor shape retention so that an EO-PO copolymer having a molecular weight of from 60 to 8000, preferably from 800 to 6000 and being in liquid form at normal temperature is used.

Next, the number of the functional groups and reaction ratio of the polyol component and polyisocyanate component will be described. It is important for a polyurethane gel exhibiting good adhesion properties and sufficient affinity for vitamins to have a three-dimensional network chain structure which is a relatively bulky molecular structure, have a segment length permitting free motion and have many linear free end molecules.

The term "good affinity for vitamins" means a property of adequately controlling the release of vitamins by dissolving them, even in not dissolving them, in the EO chain or PO chain which is a liquid segment or by bonding via an intermolecular force (by hydrogen bond or van der Waals' force). Accordingly, when the polyol and polyisocyanate are each a single compound, a combination of a bifunctional compound and a compound having at least three functional groups, or a combination of compounds each having at least three functional groups is recommended. When compounds having at least three functional groups are used in combination, however, the network chain concentration becomes too high. Without existence of very long segments in the above-described case, it is difficult to attain desirable adhesion properties because the elasticity exceeds the viscosity, and is also difficult to control an affinity for vitamins and retaining properties (release properties). Use of compounds each having roughly from 2 to 4 functional groups in combination is therefore preferred. The number of functional groups within this range enables delicate adjustment of adhesion properties and affinity for vitamins.

The reaction ratio of a polyol prepolymer and a polyisocyanate prepolymer can be controlled by a ratio of the end functional groups, that is, an OH/NCO ratio. Since remaining of an unreacted -NCO group leads to post-reaction, the OH/NCO ratio must be 1 or greater. A polyurethane gel having good adhesion properties is available at 1 ≤ OH/NCO ≤ 5. It can be imagined that at the OH/NCO ratio of 1 or greater but not greater than 5, linear segments having, at an end thereof, an OH group move freely while putting their tails out. At the ratio closer to 5, the free tails are longer and greater in number. These segments gather together to be a size suited for the exhibition of adhesion properties.

The molecular weight range of each of the polyol and polyisocyanate constituting the polyurethane gel of the cosmetic patch of the present invention differs within a wide range, depending on the kind of AO or isocyanate, the molecular form, or whether the AO is a homopolymer or copolymer. The polyurethane polyol prepolymer, the polyol and polyurethane polyisocyanate prepolymer have a molecular weight of roughly from 1400 to 10000, from 150 to 6000, and from 500 to 10000, respectively. Their molecular weights can be selected preferably from a range of roughly from 1000 to 6000, from 300 to 3000, and from 1000 to 6000, respectively.

The polyurethane gel of the cosmetic patch of the present invention is obtained by reacting the above-mentioned polyol component and the polyisocyanate component at the above-mentioned reaction ratio. The AO segment of either one of the polyol component and the polyisocyanate component is an EO chain or a PO chain having a molecular weight controlled as described above. In accordance with it, the EO chain or PO chain may be selected. When the polyurethane gel has an EO-PO copolymer as its segment, the segment having an EO/PO ratio varied depending on whether the vitamin employed is fat soluble or water soluble may be used.

When such a polyurethane gel contains a water soluble vitamin, the vitamin is retained with a good affinity in hydrophilic EO chain segments of the polyurethane gel which are in liquid form at normal temperature. When it contains a fat soluble vitamin, on the other hand, the vitamin is retained with a good affinity in hydrophobic PO chain segments which are in liquid form at normal temperature. The content of the water soluble vitamin or the content of the fat soluble vitamin can be increased or decreased by changing a molar ratio of the hydrophilic EO chain and hydrophobic PO chain.

The molar ratio of the hydrophilic EO chain and hydrophobic PO chain is adjusted preferably within a range of from 50 to 90 : from 50 to 10. When the molar ratio of the EO chain is below the above-described range, the polyurethane gel has increased hydrophobicity, leading to a decrease in the transfer and elution efficiency, to the skin, of vitamin C which is dissolved in water. When the molar ratio of the EO chain exceeds the above-described range, on the other hand, total adhesive force lowers or affinity for water becomes too high, leading to a reduction in the total release rate.

The molar ratio of the EO chain and PO chain is adjusted preferably within a range of from 50 to 10 : from 50 to 90 when the polyurethane gel contains a fat soluble vitamin. Molar ratios of the PO chain below the above-described range heighten the hydrophilicity of the polyurethane gel and lower the affinity for the fat soluble vitamin. The molar ratios of the PO chain exceeding the above-described range heightens the affinity excessively, leading to inconveniences such as reduction in a release rate.

Another polyurethane gel of the cosmetic patch of the present invention is available by reacting a polyol component with a polyisocyanate component each having, as its AO segment, the above-described EO-PO copolymer, at the above-described reaction ratio or reacting one of the polyol component and the polyisocyanate component having as its AO segment an EO-PO copolymer with the other one having as its AO segment an EO chain or PO chain.

The former polyurethane gel has only the segments of an EO-PO copolymer and it is an interpenetrated type polyurethane gel having most or all of the segments in liquid form at normal temperature. In the case of this polyurethane gel, a water soluble vitamin is retained with a good affinity in the EO portion of the segments, while a fat soluble vitamin is retained with a good affinity in the PO portion of the segments. This polyurethane gel can therefore contain both water soluble vitamin and fat soluble vitamin. By adjusting the EO : PO molar ratio to be similar to that described above, it is possible to give the polyurethane gel with a good affinity for a water soluble vitamin or fat soluble vitamin and a good release rate to the skin.

The latter polyurethane gel has both the EO-PO copolymer segments and EO or PO chain segments and it is an interpenetrated type polyurethane gel having most or all of the segments in liquid form at normal temperature. In the case of this polyurethane gel, a water soluble vitamin is retained with a good affinity in the EO portion of the copolymer segments and EO chain segments, while a fat soluble vitamin is retained with a good affinity in the PO portion of the copolymer segments and the PO chain segments. Accordingly, this polyurethane gel can also contain both water soluble vitamin and fat soluble vitamin. By adjusting the EO : PO molar ratio to be similar to that described above, it is possible to give the polyurethane gel with a good affinity for a water soluble vitamin or fat soluble vitamin and a good release rate to the skin.

As described above, either one of a water soluble vitamin and a fat soluble vitamin can be incorporated in the polyurethane gel of the cosmetic patch of the present invention. As the water soluble vitamin, any of the conventionally known ones such as vitamin B₁ (thiamine), vitamin B₂ (riboflavin), vitamin B₆ (pyridoxine), vitamin B₁₂ (cobalamin), vitamin C (L-ascorbic acid), niacin, pantothenic acid, folic acid and biotin, and derivatives thereof are usable, while as the fat soluble vitamin, any of the conventionally known ones such as vitamin A (retinol), vitamin D (calciferol), vitamin E (tocopherol) and vitamin K (phylloquinone, menaquinone) or derivatives thereof are usable.

Although no particular limitation is imposed on the content of the vitamin in the polyurethane gel, it is proper to adjust the content to fall within a range of from 2 to 20 wt%, with a range of from 3 to 10 wt% being preferred. When the vitamin content is less than 2 wt%, effects of the vitamin for whitening, pigmentation control, skin anti-aging, skin roughness prevention and the like cannot be exhibited fully. The vitamin contents exceeding 20 wt%, on the other hand, do not bring about proportional effects and on the contrary, they lead to inconveniences such as irritation causing skin damage. Even if such a large amount of an acid such as vitamin C is added, it does not deteriorate the polyurethane gel unless a large amount of an acidic substance is formed by the hydrolysis of Vitamin C through the intervention of water.

A water soluble vitamin in crystal form at normal temperature may be incorporated in the polyurethane gel after dissolved, together with a humectant, in a sufficient amount of water, while an oil soluble vitamin in crystal form at normal temperature may be incorporated after dissolved in a fatty oil or the like with an aid of an oil additive. A vitamin in liquid form at normal temperature may be incorporated as is or after dilution.

Examples of the humectant include ethylene glycol, diethylene glycol, glycerin, polyglycerin, plant collagen, cellulose and derivatives thereof, while examples of the oil additive include esters between glycerin and a fatty acid (for example, isostearyl glycerin) and hydroxyethyl isostearyloxy diisopropanolamine.

The above-described vitamin may be added during the reaction between the polyol component and the polyisocyanate component to form the polyurethane gel, or may be added after the formation of the polyurethane gel. The polyurethane gel of the cosmetic patch of the present invention does not contain water different from the conventional acrylic hydrous gel so that it absorbs and contains a solution of the vitamin very quickly even if the solution is added after the formation of the polyurethane gel as in the latter case.

Particularly in the case of vitamin C which degrades and deteriorates rapidly owing to considerably easy progress of oxidation and hydrolysis, it is important to retain vitamin C in the gel layer of the polyurethane gel which does not contain water as a solvent and therefore has a substantially no water content by adding an aqueous vitamin C solution obtained by dissolving vitamin C in water, a good solvent therefor, to the polyurethane gel and then removing water by drying; thereby preparing a stable cosmetic patch reduced in degradation and deterioration of vitamin C. The gel layer completely free of water is ideal, but complete water removal by drying or the like is very difficult. When the water content is substantially zero, that is, in a state wherein a minute amount of water as small as 1 wt% or less is contained, neither degradation nor deterioration of Vitamin C occurs so that it can be contained and retained stably in the gel layer.

The water content is preferably 0.5 wt% or less, more preferably 0.3 wt% or less. An adequate amount of water is added to impregnate the gel layer of the cosmetic patch therewith when such a cosmetic patch is applied to the skin, which makes it possible to transfer vitamin C into the skin at a good release rate by dissolving vitamin C in the gel layer.

It is needless to say that beautifying components other than Vitamin C may be incorporated in the gel layer 1 of this polyurethane gel.

The nonwoven fabric 2 is stacked over the upper surface of the gel layer 1 of the polyurethane gel and embedded in the surface layer of the gel layer 1 in order to reinforce the gel layer 1 with it and obtain the cosmetic patch 10 having sufficient strength. For example, a nonwoven fabric made of fibers of a polyolefin resin, polyacrylic resin, polyester resin or the like having a basis weight of from about 10 to 60 g/m² is preferred.

The resin film 3 is stacked over the upper surface of the gel layer 1 in order to eliminate the stickiness from the upper surface of the gel layer 1 and improve the handling ease of the cosmetic patch 10. For example, a resin film made of poly(vinyl alcohol), ethylene-vinyl acetate copolymer or polyurethane is suited. The resin film 3 preferably has a thickness of about 50 µm or less. The film exceeding this thickness lowers the flexibility of the cosmetic patch 10.

The nonwoven fabric 2 and resin film 3 may be stacked one after another over the upper surface of the gel layer 1. Alternatively, the resin film 3 having one surface laminated and reinforced with the nonwoven fabric 2 may be stacked on the upper surface of the gel layer 1 so that the nonwoven fabric 2 is located on the side of the gel layer 1.

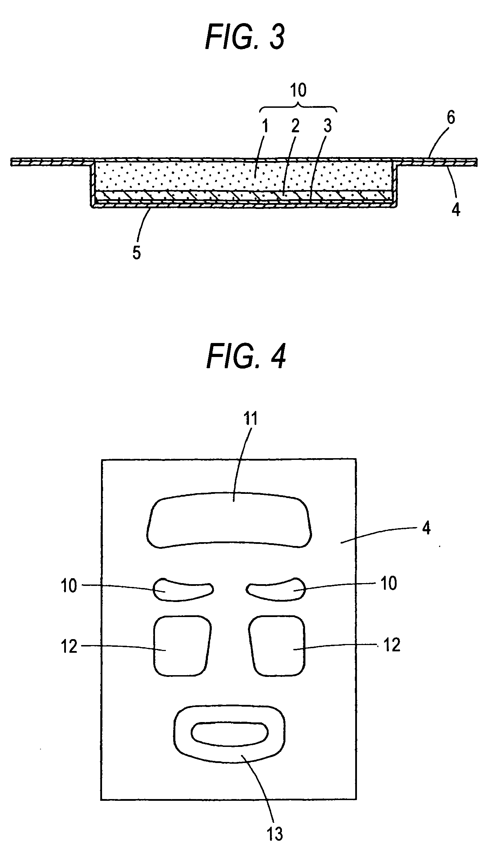

Fig. 2 is a plan view illustrating one example of a package having the above-described cosmetic patch 10 of the present invention stored therein while partially cutting away its surface cover film, and Fig. 3 is a cross-sectional view taken along a line A-A of Fig. 2.

In this package, two recesses 5 each looking like a "bird feather" are formed in a square tray 4 which has been molded from a thermoplastic resin such as polypropylene, polyethylene, poly(ethylene terephthalate) or polycarbonate. The cosmetic patches 10 of the present invention are placed in these two recesses while turning one of them upside down, and the surface cover film 6 made of a synthetic resin is placed over them to seal the tray therewith. Although the cosmetic patch 10 in this package has a shape like a "bird feather" to enable application to a face from an under-eye portion toward the tail of the eye, in short, to enable use of it as an under-eye mask, the shape can of course be changed according to a portion to be applied.

The package having the cosmetic patch 10 of the present invention stored therein can be prepared, for example, by the following processes.

One of them is to stack and lay the resin film 3 and nonwoven fabric 2 on the recess 5 of the tray 4 or lay a laminate of the resin film 3 with the nonwoven fabric 2 on the tray with the nonwoven fabric 2 up; add dropwise the above-described mixture of a polyol component and a polyisocyanate component to fill the recess 5 therewith; and react them at from 60 to 70°C to form the gel layer 1 of a polyurethane gel having the nonwoven fabric 2 and resin film 3 stacked on the bottom surface of the gel and having the nonwoven fabric 2 embedded in the surface layer of the bottom surface. A solution of a vitamin is then added dropwise to the gel layer 1 of the polyurethane gel to impregnate it with the solution, followed by attaching of a cover film 6 onto the tray 4, whereby a package having the cosmetic patch 10 stored with the upside down is prepared. In this case, a surfactant may be added to the solution of the vitamin to facilitate the penetration of the vitamin and a moisturizer into the gel layer 1. When vitamin C which is apt to undergo oxidation and hydrolysis is employed as the vitamin, the cosmetic patch 10 is made free from time-dependent discoloration or deterioration and therefore stable by adding the Vitamin C solution to the gel layer 1 and then removing water from the gel by drying.

Prior to sealing with the cover film 6, a nonwoven fabric or net may be laid over the gel layer 1. Advantages of using the nonwoven fabric or the like will be described later.

Another process is to incorporate a vitamin in the above-described polyol component by mixing it therewith or dissolving therein; add dropwise a mixture of the resulting mixture or solution with the above-described polyisocyanate component to the recess 5 of the tray 4 to fill it with the mixture; and place the nonwoven fabric 2 thereover. After the polyol component and the polyisocyanate component are reacted at from 60 to 70°C to form the gel layer 1 of the polyurethane gel having a vitamin and having the nonwoven fabric 2 stacked on and embedded in the upper surface layer, a thin resin coating for the formation of a resin film is applied to the surface of the nonwoven fabric 2 on the upper surface layer of the gel layer 1, followed by curing to stack a resin film (coating) 3 over the nonwoven fabric. The cover film 6 is then attached to the tray 4, whereby a package having the cosmetic patch 10 stored therein is prepared.

When the package thus prepared is used, the cover film 6 is peeled off and the cosmetic patch 10 of the present invention is taken out from the recess 5 of the tray 4. The cosmetic patch is applied to a face from an under-eye portion toward the tail of the eye with the resin film 3 outside. Particularly, a package having, stored therein, the cosmetic patch 10 containing Vitamin C is applied to the skin after peeling the cover film 6, adding an adequate amount of water such as cosmetic lotion to the polyurethane gel layer 1 of the cosmetic patch 10 to cause the gel layer to absorb water in accordance with the using method of the present invention to dissolve vitamin C in the gel layer 1. When the gel layer 1 has the nonwoven fabric or the like stacked thereover, dropwise addition of water to the nonwoven fabric enables uniform distribution of water all over the gel layer 1.

By the application of the cosmetic patch 10 of the present invention to the skin, a vitamin contained rich in the gel layer 1 of the polyurethane gel transfers into the skin and exhibits its effects. Even when the vitamin is vitamin C, it transfers into the skin with a good release rate by impregnating the gel layer 1 with water and dissolving vitamin C in this water upon application. The cosmetic patch has a stable adhesive force and does not peel even several hours after application, because the gel layer 1 of the polyurethane gel exhibits a good adhesive force owing to the alkylene oxide segments in liquid form at normal temperature and different from the conventional acrylic hydrous gel, its adhesive force does not undergo a change by the water content.

Fig. 4 is a plan view illustrating another example of a package having various shapes of cosmetic patches of the present invention stored in combination while omitting its surface cover film from the view.

This package is obtained by forming, in the tray 4, recesses having, stored therein, a cosmetic patch 11 to be applied to the forehead, the above-described cosmetic patches 10, 10 to be applied to portions extending from the under-eye portion to the tail of the eye, cosmetic patches 12,12 to be applied to the cheek, and a cosmetic patch 13 to be applied to a portion around the mouth respectively; preparing the cosmetic patches 10, 11, 12, 13 in the respective recesses in the above-described manner; and attaching a surface cover film (not illustrated) onto the tray 4 to seal the tray with the film. The cosmetic patches 10, 11, 12, 13 are equal in their structure, but different in their shape so as to be fit for the respective portions of the face.

Such a package is convenient because it contains cosmetic patches to be applied to the respective face portions. In addition, it can be used more widely, because compared with a cosmetic patch covering the whole face therewith, it can be used more freely regardless of the shape or size of each face.

The using method of a cosmetic patch according to the present invention relates to a using method of a cosmetic patch, of the above-described cosmetic patches, having a water soluble vitamin such as vitamin C (a description will hereinafter be made using vitamin C as an example) incorporated in the gel layer of a polyurethane gel in a dry state. Described specifically, the method comprises, just before use of a cosmetic patch which has been obtained by incorporating vitamin C, in the absence of a solvent therefor and in a dry state, in a gel layer made of an adhesive polyurethane gel having ethylene oxide segments and propylene oxide segments, or segments composed of a copolymer of ethylene oxide and propylene oxide and having most or all of these segments in liquid form at normal temperature, causing the gel layer to absorb water to dissolve vitamin C therein; and applying the cosmetic patch to the skin to transfer both vitamin C and water into the skin. Only several minutes are necessary for impregnating the gel layer with water. Even if the water absorption time is short, the gel layer of a polyurethane gel exhibits good water absorption so that it can absorb a sufficient amount of water to dissolve vitamin C therein.

When the above-described using method of the cosmetic patch is employed, effects of vitamin C can be exhibited fully, because vitamin C contained in a dry state is free from deterioration due to oxidation and hydrolysis until just before use and during use, vitamin C dissolved in water, which serves as a good solvent therefore, transfers to the skin and is absorbed therein smoothly together with water.

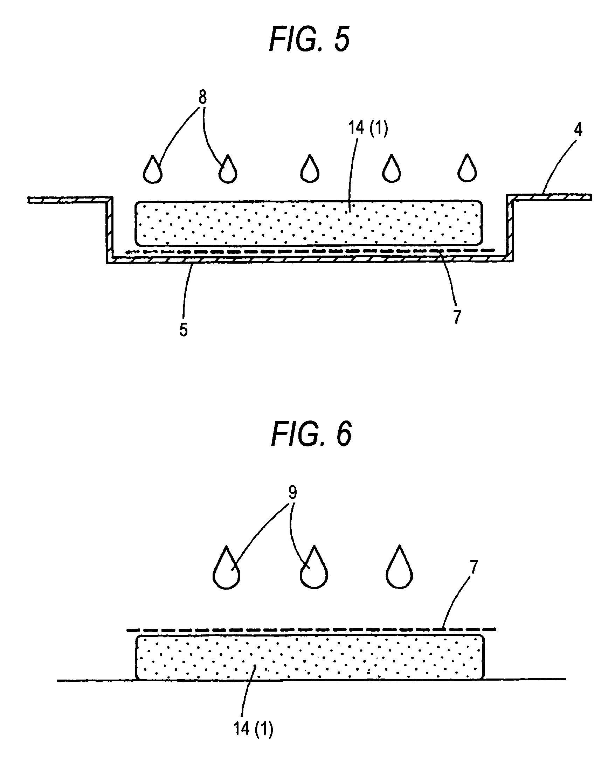

Fig. 5 is a cross-sectional view illustrating a preferred embodiment of the using method of the present invention. In the using method of the present invention, as illustrated in this diagram, it is preferred to stack a net made of a synthetic resin over the bottom surface of a cosmetic patch 14, store the resulting cosmetic patch in a concave body, for example, a tray 4 having a recess 5, add water 8 to impregnate the patch with water, remove the net 7 and apply the resulting cosmetic patch 14 to the skin. By the addition of water 8 after the net 7 made of a synthetic resin is stacked over the bottom surface of the cosmetic patch 14, water 8 is absorbed from the upper surface of the cosmetic patch 14 and at the same time, water 8 runs toward the bottom surface side of the cosmetic patch 14 along the net 7. Uniform absorption of water 8 in the gel layer also from the whole bottom surface enables uniform dissolution of vitamin C, which has been contained in the gel layer, in water and smooth transfer and absorption of the resulting solution into the skin.

As the concave body, use of the tray 4 having the recess 5 a little greater than the cosmetic patch 14 is desired. Use of such a tray 4 facilitates running of water 8 through the space between the inside surface of the recess 5 and the cosmetic patch 14 toward the bottom surface side.

The net 7 made of a synthetic resin is disposed in order to enable running of the water 8 toward the bottom surface of the cosmetic patch 14 and uniform penetration and absorption of it from the bottom surface into the gel layer 1 of the cosmetic patch; and during dropwise addition of an aqueous solution of vitamin C to the gel layer 1 of the cosmetic patch to impregnate the gel layer with the aqueous solution as described later, to spread the aqueous solution all over the surface of the gel layer 1 to which the aqueous solution is added dropwise and enable uniform absorption of it.

Accordingly, a net made of a synthetic resin which has a good affinity for water or an aqueous solution of Vitamin C and does not absorb vitamin C, for example, a net having a mesh size of from about 30 to 100 obtained by crossing monofilaments made of a nylon resin having a thickness of from about 100 to 300 µm is preferred. Since there is a fear of vitamin C being trapped between twisted yarns constituting a net, a net made of monofilaments free from such a fear is preferred.

As illustrated in Fig. 6, this net 7 is preferably stacked over the upper surface of the gel layer 1 in advance when the aqueous solution 9 of vitamin C is added dropwise to the gel layer 1 of the cosmetic patch 14 to impregnate it with the aqueous solution. By the dropwise addition of the aqueous solution 9 of vitamin C while stacking the net 7 over the gel layer, the aqueous solution 9 of vitamin C spreads uniformly all over the upper surface of the gel layer 1 along the net 7 and is absorbed in the gel layer 1. In addition, it prevents dropping of the aqueous solution 9 of vitamin C from the gel layer 1. When water, which serves as a solvent, is removed by vacuum drying of the gel layer 1, the cosmetic patch 14 having, to the upper surface thereof, the net 7 temporarily adhered and containing a large amount of vitamin C on the upper surface side of the gel layer 1 can be obtained.

When the cosmetic patch 14 equipped with a net is turned upside down as illustrated in Fig. 5, placed in the recess 5 of the tray 4, stored while hermetically sealing it with a cover film (not illustrated), and is added with water 8 after peeling of the cover film just before use, the water 8 runs toward the bottom surface side of the cosmetic patch 14 along the net 7 and is absorbed uniformly in the gel layer 1 from the bottom surface side rich in vitamin C, whereby vitamin C can be dissolved in the water in a short time and the net 7 can be peeled from the gel layer 1 easily. After the net is peeled and the bottom surface of the cosmetic patch 14 having a high Vitamin C concentration is applied to the skin, Vitamin C exhibits its effects fully as a result of transfer and release of a large amount of vitamin C to the skin.

Neither the above-mentioned nonwoven fabric 2 nor the resin film 3 is stacked over the upper surface of the gel layer 1 in the cosmetic patch 14 illustrated in Fig. 5. It is needless to say, however, that they may be stacked. Even if the nonwoven fabric 2 or resin film 3 is stacked, there occurs no problem, because water thus added runs toward the bottom surface of the gel layer 1 and is absorbed therein uniformly. When in Fig. 6, the aqueous solution 9 of vitamin C is added dropwise to and is absorbed in the gel layer 1, an aqueous solution of vitamin C added with an adequate amount of an alcohol may be used as described above. In this case, the addition of the alcohol lowers the surface tension of the aqueous solution 9 of vitamin C and disturbs repelling of it, which facilitates the absorption of it into the gel layer 1 further.

The cosmetic patch of the present invention which contains a water soluble vitamin other than vitamin C in a substantially water-free adhesive polyurethane gel having hydrophilic alkylene oxide segments and hydrophobic alkylene oxide segments and having most or all of these segments in liquid form at normal temperature can also be used for the above-described cosmetic patch package.

The package (cosmetic patch package) of the present invention having, stored therein, a cosmetic patch containing a water soluble vitamin will next be described by some specific examples. It should however be borne in mind that the present invention is not limited to or by them.

### (1) Package Example 1

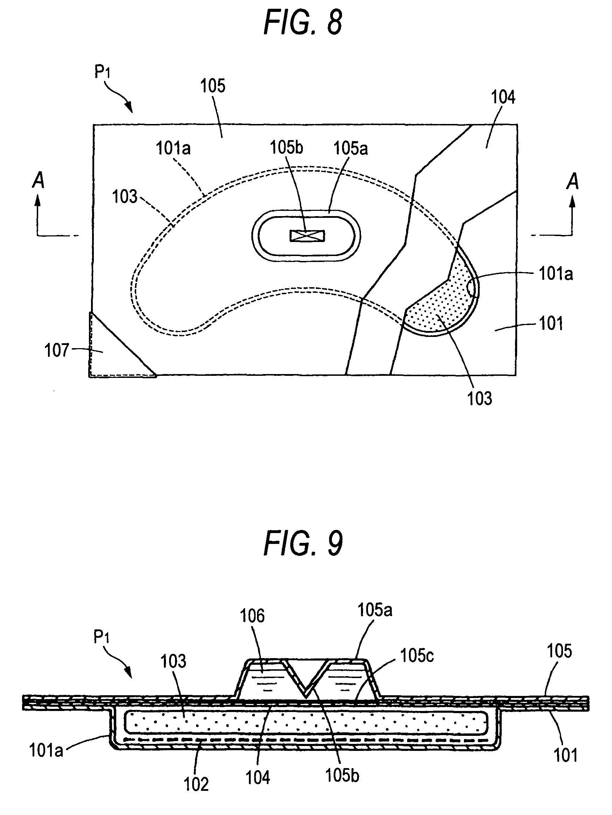

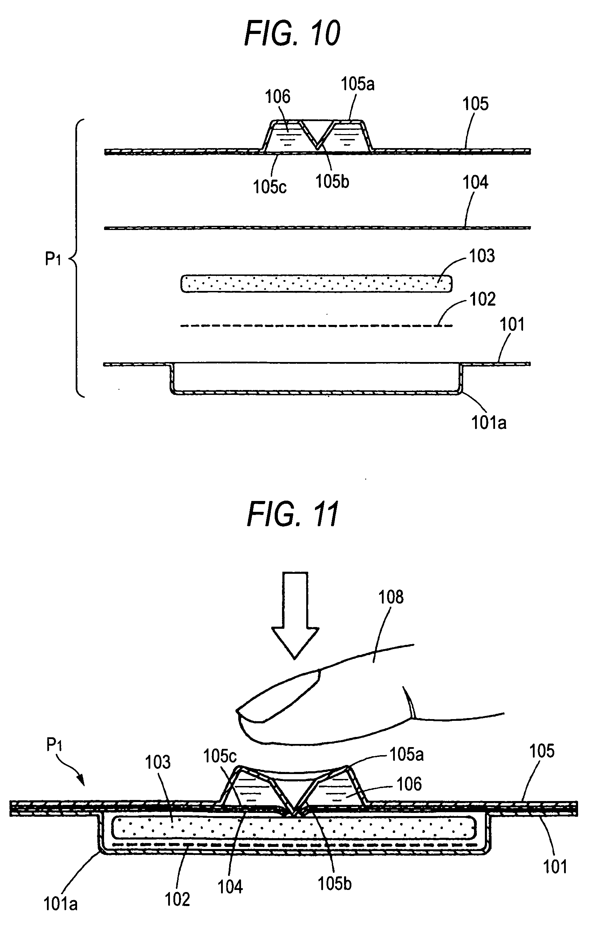

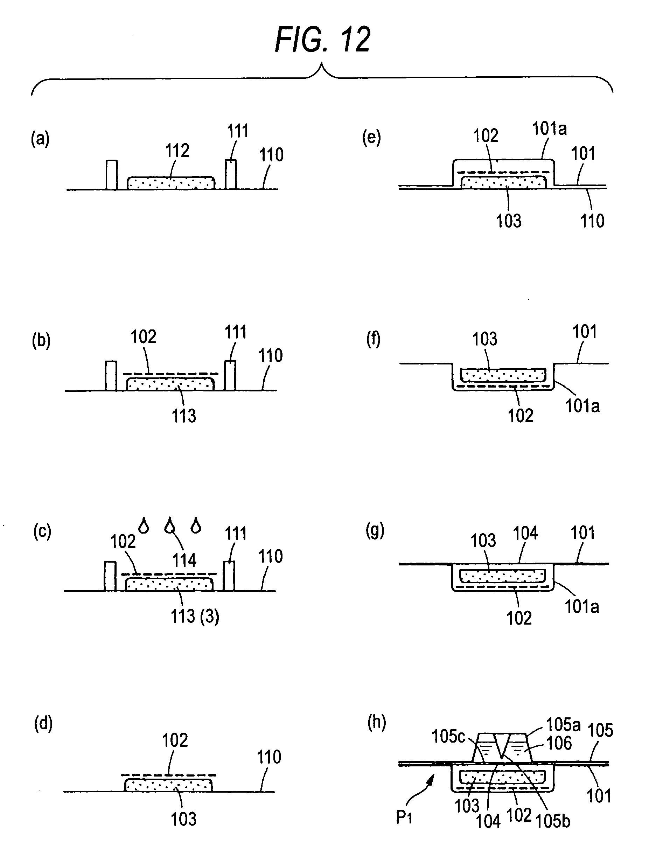

Fig. 8 is a partial cutaway plain view of a cosmetic patch package according to one embodiment of the present invention; Fig. 9 is a cross-sectional view taken along a line A-A of Fig. 8; Fig. 10 is an exploded cross-sectional view of the cosmetic patch package; Fig. 11 is an explanatory view of a using method of the cosmetic patch package; and Fig. 12 is an explanatory view illustrating one example of a manufacturing method of the cosmetic patch package.

This cosmetic patch package P₁ is, as illustrated in Fig. 10, composed of a tray 101 made of a synthetic resin, a net 102 made of a synthetic resin, a cosmetic patch 103 made of a polyurethane gel containing a water soluble vitamin (in particular, vitamin C (ascorbic acid)), a cover film 104 having a weak tear strength, and a cover unit 105 having a water reservoir 105a.

The tray 101 is a molded product made of a thermoplastic resin such as polypropylene, polyethylene, polyethylene terephthalate or polycarbonate and it has a rectangular planar shape as illustrated in Fig. 8. It has, at the center thereof, a storing recess 101a for storing the cosmetic patch 103 therein.

In this Embodiment, the storing recess 101a has a curved planar shape corresponding to the shape of the cosmetic patch 103 so as to store the cosmetic patch 103 having a curved planar shape to be used for a portion of the face from an under-eye portion to the tail of the eye, in short, to be used as an under-eye mask.

The net 102 made of a synthetic resin is laid over the bottom surface of this storing recess 101a and over this net 102, the cosmetic patch 103 is stacked. Thus, the net and the cosmetic patch are stored in this storing recess. This net 102 is set in order to enable running of water, which has entered into the storing recess 101a from the water reservoir 105a of the cover unit 105, toward the bottom surface of the cosmetic patch 3 and also enable uniform penetration and absorption of the water into the segmented polyurethane gel of the cosmetic patch 103 from the bottom surface at the time of use; and during dropwise addition of an aqueous solution of vitamin C to the segmented polyurethane gel of the cosmetic patch 103 to impregnate it with the aqueous solution as described later, to spread the aqueous solution all over the surface of the polyurethane gel to which the aqueous solution is added dropwise and enable uniform absorption of it.

As the net 102 made of a synthetic resin, a net having a good affinity for water or an aqueous solution of Vitamin C and does not absorb vitamin C and having a mesh size of from about 30 to 100 obtained by crossing monofilaments made of a nylon resin having a thickness of from about 100 to 300 µm is preferred as described above.

The cosmetic patch 103 has a water soluble vitamin (in particular, which may be typically described as "typical Vitamin C") incorporated in an adhesive polyurethane gel. In this embodiment, it has a curved planar shape as indicated by a dotted line in Fig. 8 so that it can be applied, as an under-eye mask, to a face portion from an under-eye portion to the tail of an eye. Although no particular limitation is imposed on the thickness of the cosmetic patch, thickness of about from 0.5 to 2.0 mm is preferred. When the cosmetic patch is thinner than 0.5 mm, the vitamin C content decreases and there is a fear of beautifying effects becoming insufficient. Even if the thickness exceeds 2.0 mm, on the other hand, the effect thus available is not proportional to the increase, leading to waste of the material. It is preferred to reinforce the cosmetic patch 103 by embedding a nonwoven fabric (not illustrated) in the surface layer of the segmented polyurethane gel.

It is also preferred to make the cosmetic patch 103 strong and stickiness-free by attaching a laminate sheet of a nonwoven fabric and a synthetic resin film on the surface layer of this segmented polyurethane gel. For example, a nonwoven fabric made of fibers of a polyolefin resin, polyacrylic resin, polyester resin or the like having a basis weight of from about 10 to 60 g/m² is preferred. As the synthetic resin film, that made of polyvinyl alcohol, ethylene-vinyl acetate copolymer or urethane and having a thickness of 50 µm or less is preferred.

The polyurethane gel of this cosmetic patch 103 is a substantially water-free adhesive gel and most or all of its segments are in liquid form at normal temperature. More specifically, it has hydrophilic ethylene oxide (EO) segments and hydrophobic propylene oxide (PO) segments, or has segments composed of a copolymer between hydrophilic ethylene oxide (EO) and hydrophobic propylene oxide (PO).

Vitamin C is, after its aqueous solution is impregnated in the above-described polyurethane gel, dried under reduced pressure, whereby it is contained and retained in the EO segment portion of the substantially water-free polyurethane gel or in the EO portion of the copolymer segment with an affinity. Vitamin C can therefore be contained and retained in the gel over a prolonged period without causing a time-dependent deterioration due to oxidation and hydrolysis. In addition, without generation of an acidic substance, deterioration of the polyurethane gel does not occur. Vitamin C may be absorbed in the polyurethane gel after dissolved in a mixed solvent of water and an alcohol such as ethanol. Addition of such a solution of vitamin C in a mixed solvent to the polyurethane gel improves its absorption, because the surface tension of the solution lowers, which disturbs repelling of it.

The molar ratio of the EO segments and PO segments of the polyurethane gel is adjusted preferably within a range of from 50 to 90 : from 50 to 10. When the molar ratio of the EO segments is below the above-described range, the polyurethane gel has increased hydrophobicity, leading to a decrease in the transfer and release efficiency, to the skin, of vitamin C which is dissolved in water at the time of use. When the molar ratio of the EO segments exceeds the above-described range, total adhesive force of the polyurethane gel lowers or affinity for water becomes too high, leading to a reduction in the total release rate of vitamin C. Similarly, it is preferred to adjust the molar ratio of EO and PO of the copolymer segments to from 50 to 90 : from 50 to 10.

The polyurethane gel is similar to that described above so that a detailed description on it is omitted.

The cover film 104 for covering the storing recess 101a of the tray 101 therewith serves to hermetically seal the storing recess 101a to prevent moisture absorption of the polyurethane gel of the cosmetic patch 103 stored in the storing recess 101a and it is stacked on the upper surface of the tray 101.

As such a cover film 104, a metal foil which has no air permeability and has a weak tear strength permitting easy opening at the time of use such as aluminum foil (having a thickness of from about 5 to 50 µm) or a metal deposited film such as aluminum-deposited film (having a thickness of from about 10 to 50 µm) is preferred. When an external force is applied to an aluminum foil, for example, by pushing, it can be easily torn to open the tray, while a metal deposited film is perforated by a V-shaped protrusion 105b, which will be described later, to open the tray.

The cover unit 105 stacked over the tray 101 is obtained by molding a thermoplastic synthetic resin such as poly(vinyl chloride) resin and it has, in the vicinity of the center thereof, the water reservoir 105a which has an oblong planar shape and is convexly curved. On the upper surface of this water reservoir 105a, the V-shaped protrusion 105b having a pointed end is provided in a downward direction.

In this water reservoir 105a, water 106 to be fed to the storing recess 101a of the tray 101 is poured and the water 106 is hermetically sealed in the water reservoir with a cover film 105c which is stacked over the bottom surface of the cover unit 105. This cover film 105c is made of a similar material to that used for the above-described cover film 104 of the tray 101 and an aluminum foil or aluminum-deposited film which has tear strength weak enough to permit easy opening at the time of use is used.

The cover unit 105 may be stacked over and attached onto the tray 101 in advance, or may be stacked over the tray 101 at the time of use. In this case, it is desired to temporarily joint the tray 101 and the cover unit 105 by clipping the corner portions of them with a right triangular bracket clip 107. When the cover unit 105 is stacked over the tray 101 and they are jointed temporarily, the water reservoir 105a of the cover unit 105 is located above the center portion of the storing recess 101a of the tray 101.

When the cosmetic patch package P₁ having the constitution as described above is used for under-eye pack, an external force is applied by pushing the water reservoir 105a of the cover unit 105 downward with a fingertip 108 as illustrated in Fig. 11. The protrusion 105b of the water reservoir 105a then breaks through the cover film 105c on the bottom surface of the water reservoir and the cover film 104 on the upper surface of the tray and the water 106 is poured into the storing recess 101a of the tray 101 from the breakage. The water 106 then penetrates from the upper surface of the cosmetic patch 103 into the polyurethane gel and at the same time, it runs toward the bottom surface side of the cosmetic patch 103 and extends over the whole surface thereof along the net 102. Water thus penetrates and is absorbed in the polyurethane gel uniformly also from the bottom surface. Vitamin C contained in the polyurethane gel dissolves in the water thus absorbed therein.

When the cosmetic patch 103 taken out from the storing recess 101a of the tray 101 after removal of the cover unit 105 and the cover film 104 is applied to an under-eye portion of the face by making use of the adhesive force of the polyurethane gel, vitamin C thus dissolved in water transfers to the skin from the polyurethane gel and is absorbed in the skin smoothly. There, it exhibits effects of vitamin C such as whitening, pigmentation prevention, and antioxidant ones.

In the cosmetic patch package P₁ of the present invention, the cosmetic patch 103 contains vitamin C in the substantially water-free polyurethane gel in a dry state so that vitamin C can be incorporated and retained stably without causing oxidation and hydrolysis and moreover, discoloration or quality change attributable thereto, and the polyurethane gel undergoes neither quality change nor deterioration which will otherwise occur by the generation of an acidic substance. Since the polyurethane gel is impregnated with the water 106 from the water reservoir 105a of the cover unit 105 and vitamin C is dissolved in the water at the time of use, transfer and release of vitamin C together with water to the skin are smoothly and promptly carried out when the cosmetic patch 103 is applied to the skin. The cosmetic patch package P₁ of the present invention is thus an epoch-making product capable of overcoming the above-described trade-off, that is, vitamin C is hydrolyzed with water but water is necessary for effective release of vitamin C.

With reference to Fig. 12, a manufacturing method of the cosmetic patch package P₁ will next be described.

As illustrated in Fig. 12(a), a frame 111 is placed on a mold release film 110 and inside of the frame, a mixture 112 of the polyol component and the polyisocyanate component is cast. The mixture 112 is reacted by heating to obtain a segmented polyurethane. A net 102 is laid over the resulting polyurethane gel 113 as illustrated in Fig. 12(b), followed by the dropwise addition of an aqueous solution 114 of Vitamin C downward as illustrated in Fig. 12(c).

When the aqueous solution of vitamin C is added dropwise downward to the net 102, the aqueous solution of vitamin C extends all over the upper surface of the polyurethane gel 113 along the net 102 and is absorbed uniformly in the gel. After completion of the absorption, the frame is removed as illustrated in Fig. 12(d) and water is removed by drying under reduced pressure, whereby a cosmetic patch 103 is obtained.

A tray 101 is laid over the cosmetic patch 103 and net 102 as illustrated in Fig. 12(e). The tray 101 is then turned upside down as illustrated in Fig. 12(f) to store the cosmetic patch 103 and net 102 in a storing recess 101a. As illustrated in Fig. 12(g), a cover film 104 such as aluminum foil is stacked over the tray 101 and the cosmetic patch 103 and the net 102 are hermetically sealed in the storing recess 101a. As illustrated in Fig. 12(h), a cover unit 105 obtained by filling a water reservoir 105a with water 106 and hermetically sealing with a cover film 105c is laid over the tray 101, whereby the cosmetic patch package P₁ is prepared.

In order to heighten the strength of the cosmetic patch 103 by stacking a nonwoven fabric over the surface layer of the polyurethane gel of the cosmetic patch 103, it is only necessary, in Fig. 12(a), to lay the nonwoven fabric over the slip film 110 prior to the casting of the mixture 112 of the polyol component and the polyisocyanate component and then, cast the mixture 112 over the nonwoven fabric. The nonwoven fabric stacked over the surface layer of the polyurethane gel makes it possible to uniformly impregnate the whole gel layer 101 with water when the water reservoir of the cover unit is broken to cause water penetration and absorption in the polyurethane gel. Instead of the nonwoven fabric, a nonwoven fabric laminated with a synthetic resin film may be used. In this case, stickiness can be eliminated from the surface of the cosmetic patch 3.

### Package Example (2)

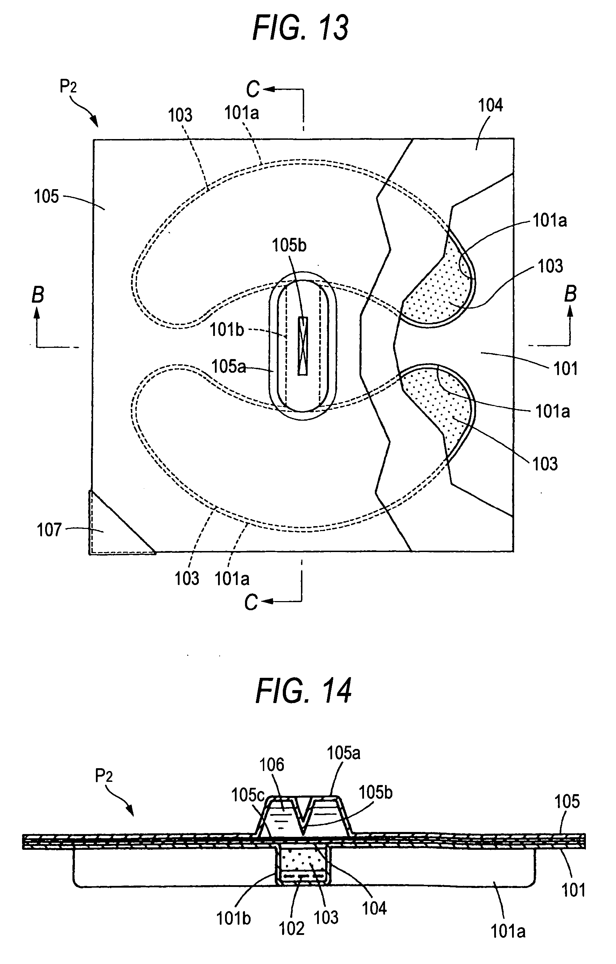

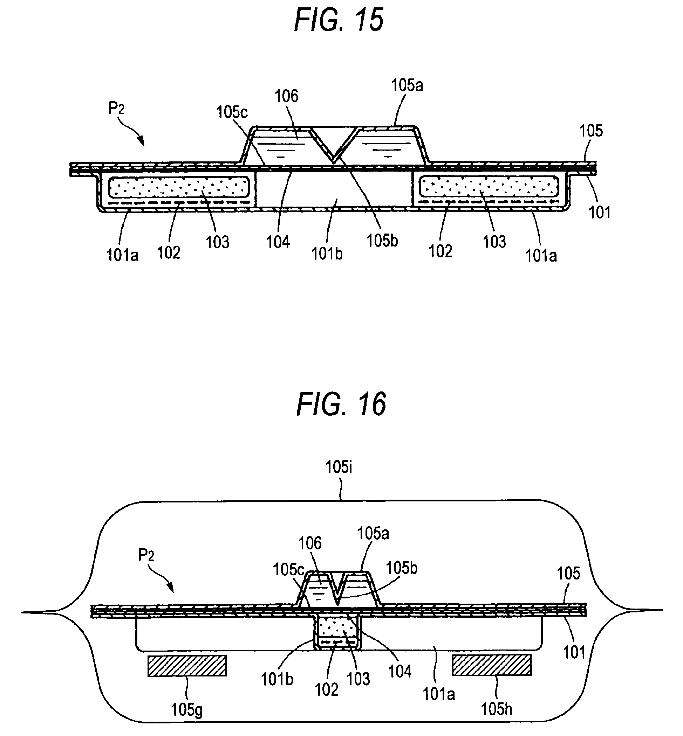

Fig. 13 is a part cutaway plan view of a cosmetic patch package according to another embodiment of the present invention; Fig. 14 is a cross-sectional view taken along a line B-B of Fig. 13; and Fig. 15 is a cross-sectional view taken along a line C-C of Fig. 13.

This cosmetic patch package P₂ has two storing recesses 101a, 101a formed in the tray 101. In the storing recesses 101a, 101a, the cosmetic patches 103, 103 having vitamin C incorporated in a polyurethane gel in a dry state and the nets 102, 102 are stacked one after another, respectively. The tray 101 has a connecting channel 101b for connecting these two storing recesses 101a, 101a and a cover unit 105 is laid over the tray 101 so that the water reservoir 105a of the cover unit 105 is located above this connecting channel 101b. The cosmetic patch package P₂ is similar to the above-described cosmetic patch package P₁ except for the above-described constitution so that in FIGS. 13 to 15, like members will be identified by like reference numerals and overlapping descriptions will be omitted.

The cosmetic patch package P₂ having such a constitution is also able to contain and retain vitamin C in the polyurethane gel of the cosmetic patch 103 stably without causing oxidation and hydrolysis of it. When an external force is applied to the water reservoir 105a of the cover unit 105 by pushing downward with a finger at the time of use, the protrusion 105b of the water reservoir 105a of the tray 101 breaks the cover film 105c on the bottom surface of the water reservoir and the cover film 104 on the upper surface of the tray and from this breakage, water 106 flows into the two storing recesses 101a, 101a through the connecting channel 101b of the tray 101 and is absorbed in the polyurethane gel of each cosmetic patch 103. After removal of the cover unit 105 and cover film 104, the two cosmetic patches 103, 103 are applied to under-eye portions of the face by making use of the adhesive force of the polyurethane gel. Vitamin C which has dissolved in water transfers from the polyurethane gel to the skin and is absorbed smoothly and promptly. Thus, the effects of vitamin C such as whitening, pigmentation prevention and antioxidant ones are exhibited. This cosmetic patch package P₂ is convenient because water 106 can be absorbed in the two cosmetic patches 103, 103 simultaneously only by tearing one water reservoir 105a as described above.

In the above-described embodiment, the polyurethane gel is stored in the storing recess 101a of the tray 101 and hermetically sealed with the cover film 104 in order to prevent moisture absorption. It is preferred to put such a cosmetic patch package P₂ together with an oxygen absorber and/or desiccant in an outer bag having gas barrier properties. A package used for such an embodiment is illustrated in Fig. 16. Fig. 16 is a cross-sectional view illustrating an outer bag 105i, having a moisture-proof material such as aluminum deposited or laminated thereon, in which the package P₂ as illustrated in Fig. 14 having the cosmetic patch stored therein, an oxygen absorber 105g and a desiccant 105h are stored together. Since the outer bag 105i contains the cosmetic patch package P₂ in such a manner, oxygen and water are removed from the outer bag 105i and the cosmetic patch is blocked doubly from the outside environment, making it possible to prevent oxidation and hydrolysis of vitamin C or deterioration of polyurethane gel. This cosmetic patch package is similar to that of Fig. 14 except for the above-described constitution so that like members are identified by like reference numerals and overlapping descriptions are omitted.

### Package Example (3)

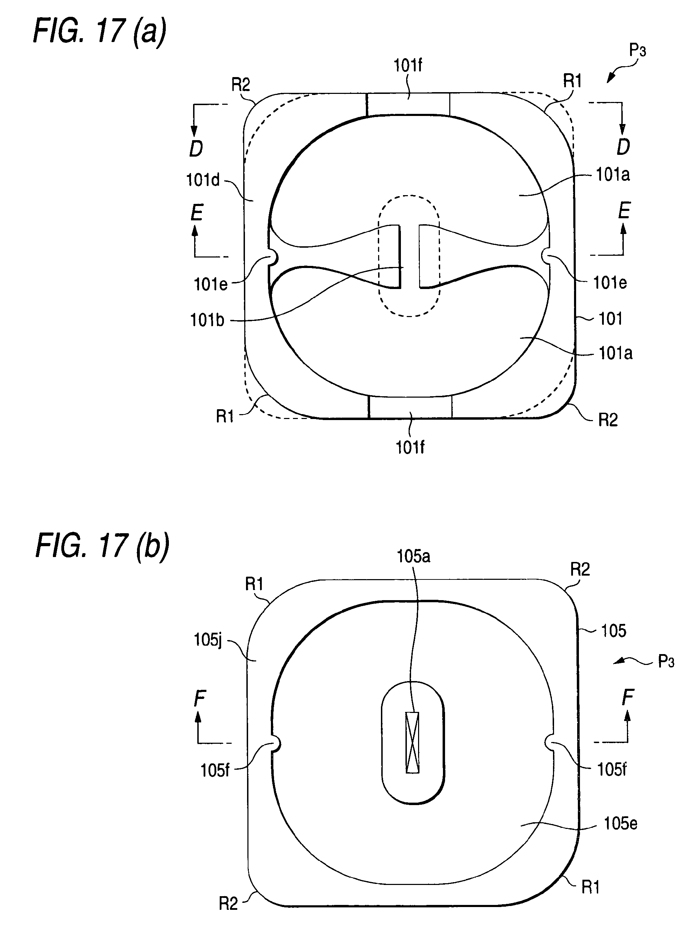

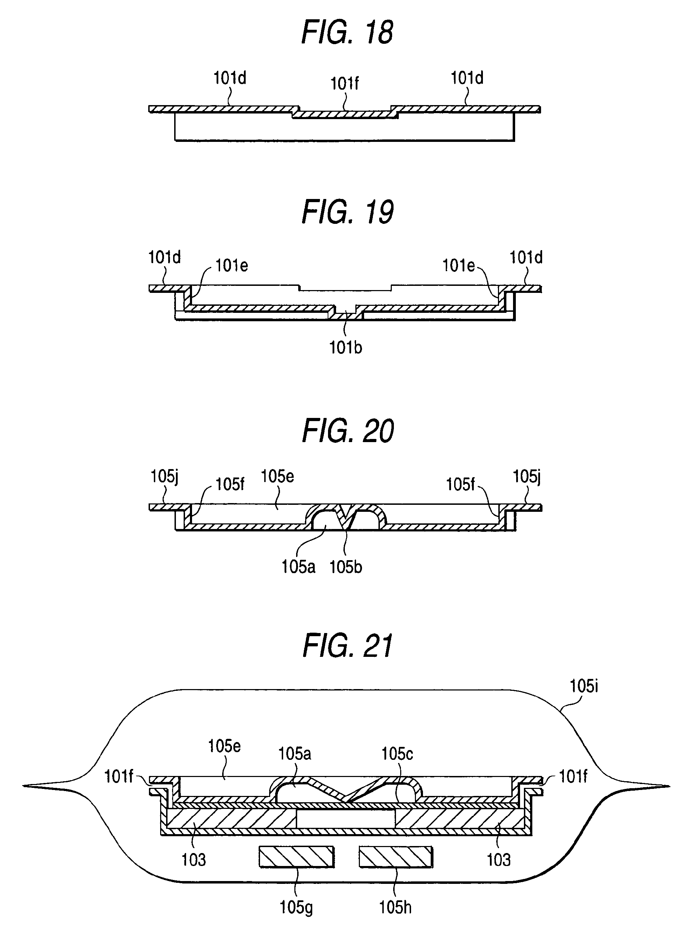

Fig. 17(a) is a plan view illustrating a tray of a cosmetic patch package according to a further embodiment of the present invention, Fig. 17(b) is a plan view of a cover unit of this cosmetic patch package; Fig. 18 is a cross-sectional view taken along a line D-D of Fig. 17(a); Fig. 19 is a cross-sectional view taken along a line E-E of Fig. 17(a); and FIG. 20 is a cross-sectional view taken along a line F-F of Fig. 17(b).

Similar to the cosmetic patch package P₂, this cosmetic patch package P₃ has, in the tray 101 thereof, two storing recesses 101a, 101a and also a connecting channel 101b for connecting these recesses. The storing recess 101a is able to store therein a cosmetic patch having vitamin C incorporated in the polyurethane gel thereof in a dry state. In the tray 101 as illustrated in Fig. 17(a) different from the above-described package, two bulges 101e, 101e protruding inward are formed on the inner side surface of two collar portions 101d, which are opposite to each other, existing at the periphery of the storing recess 101a and at the same time, a step difference is formed, as an opening 101f, by denting the collar portion 101d on the other side surfaces opposite to each other.

On the other hand, the cover unit 105 as illustrated in Fig. 17(b) has a water reservoir 105a similar to that of the cosmetic patch package P₂. This cover unit 105 is not a flat sheet, but is designed to fit in the storing recess 101a of the tray 101 by forming a dent 105e at the center of the cover unit and at the same time, a recess 105f to fit with the bulge 101e of the tray 101 is formed.

The cover unit 105 cannot be fitted in the tray 101 unless the bulge 101e of the tray 101 is fitted in the recess 105f of the cover unit 105. When they are fitted, the position and direction of the water reservoir 105a of the cover unit 105 are determined and the water reservoir lies right above the connecting channel 101b of the tray 101 in the same direction. As a result, water from the water reservoir 105a can be fed to the connecting channel 101b without fail.

When the cover unit 105 is fitted in the tray 101, the passage 101f of the tray 101 forms a space with the collar portion 105j of the cover unit 105 and is connected to the outside. This passage 101f permits air flow in and out of the tray 101.

The tray 101 and cover unit 105 are not formed, at the four corners thereof, with equal curvatures. Curvatures R1, R1 of two corners of the tray 101 opposite to each other are different from curvatures R2, R2 of the other two corners of the tray 101 opposite to each other. Curvatures R1, R1 of two corners of the cover unit 105 opposite to each other are, on the other hand, different from curvatures R2, R2 of the other two corners. When the cover unit 105 is fitted in the tray 101 in a fixed direction, the two corners R1 of the tray 101 overlap with the two corners R2 of the cover unit 105, while the two corners R2 of the tray 101 overlap with the two corners R1 of the cover unit 105.

As a result, the corners R1 of the cover unit 105 which have smaller curvatures protrude from the corners R2 of the tray 101 which have greater curvatures. The cover unit 105 can therefore be peeled from the tray 101 easily by having these protrusions in hands. All the four corners of the tray and cover unit may be formed with different curvatures. In this case, a protrusion appears irrespective of the overlapping direction of the tray and cover. Alternatively, a protrusion is caused to appear by differentiating the peripheral shape between the tray and cover and overlapping them each other.

As illustrated in Fig. 16, it is necessary to put the cosmetic patch package P₃ of such an embodiment in an outer bag 105i having gas barrier properties, together with an oxygen absorber 105g and a desiccant 105h. The cosmetic patch package P₃ put in the outer bag 105i leads to the inside of the outer bag 105i through the passage 101f of the tray 101 so that oxygen and water removing effects of the oxygen absorber 105g and desiccant 105h put in the outer bag 105i extend even inside of the tray 101, whereby deterioration in the quality of the cosmetic patch package P₃, in other words, oxidation and hydrolysis of vitamin C and deterioration of polyurethane gel can be suppressed furthermore.

In this Embodiment, the tray and cover unit are equipped with bulge and recess, respectively. On the contrary, the tray and cover unit may be equipped with recess and bulge, respectively. Their shapes are not limited to those illustrated in the diagram.

### Package Example (4)

Fig. 21 is a partial cutaway plan view of a cosmetic patch package according to a still further embodiment of the present invention.

Similar to the cosmetic patch package P₂, this cosmetic patch package P₄ has two storing recesses 101a, 101a formed in the tray 101. In these storing recesses 101a, 101a, the cosmetic patches 103, 103 each having, in the polyurethane gel thereof, vitamin C incorporated in a dry state and the net 102 are stacked one after another and stored, respectively.

This tray 101 however has no connecting channel for connecting these two storing recesses 101a, 101a. A cover unit 105 having two water reservoirs 105a, 105a is laid over the tray 101 and these two water reservoirs 105a, 105a are each located above the vicinity of the center of the storing recess 101a. This cosmetic patch package is similar to the above-described cosmetic patch package except for the above-described constitution so that in Fig. 20, like members are identified by like reference numerals and overlapping descriptions are omitted.

In such a cosmetic patch package P₃, the cosmetic patch 103 is able to incorporate vitamin C in its polyurethane gel stably without causing oxidation and hydrolysis of it. After these two water reservoirs 105a, 105a are broken by pushing with a finger and the polyurethane gels of the cosmetic patches 103, 103 are impregnated with water 106, 106, the cosmetic patches 103, 103 are applied to both under-eye portions of the face. Then, vitamin C, together with water, transfers to the skin and effects of vitamin C such as whitening, pigmentation prevention and antioxidant effects are exhibited. Although the two water reservoirs 105a, 105a must be broken, this cosmetic patch package P₄ is advantageous because a predetermined amount of water can be supplied to the two storing recesses 101a, 101a without leakage.

### Package Example (5)

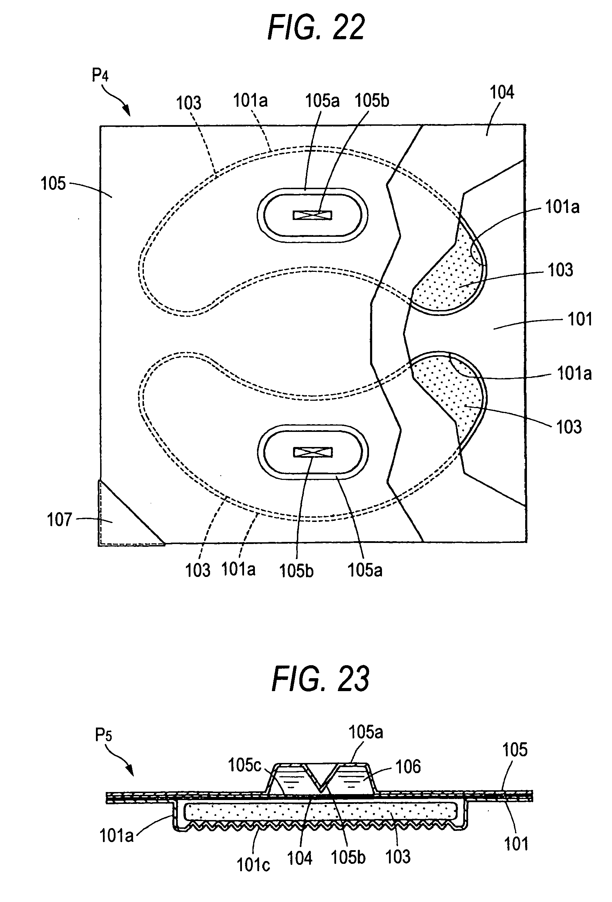

Fig. 22 is a cross-sectional view of a cosmetic patch package according to a still further embodiment of the present invention.

In this cosmetic patch package P₅, small irregularities 101c are formed on the bottom surface of the storing recess 101a of the tray 101. This storing recess 101a contains therein only the above-described cosmetic patch 103 having vitamin C incorporated in the polyurethane gel in a dry state. This cosmetic patch package is similar to the cosmetic patch package P₁ except for the above-described constitution so that in Fig. 22, like members are identified by like reference numerals and overlapping descriptions are omitted.

This cosmetic patch package P₅ exhibits similar effects and advantages to those of the cosmetic patch package P₁. Even if a net is not laid over the storing recess 101a of the tray 101, water 106 running into the storing recess 101a of the tray 101 by pushing and breaking the water reservoir 105a of the cover unit 105 enters on the bottom surface side of the cosmetic patch 103 through spaces (valleys) of the irregularities 101c on the bottom surface. The cosmetic patch 103 can therefore absorb water uniformly even from its bottom surface. Since the cosmetic patch package according to this embodiment is not equipped with a net, the number of parts can be reduced.

### Package Example (6)

Fig. 23 is an exploded cross-sectional view illustrating a cosmetic patch package according to a still further embodiment of the present invention.

This cosmetic patch package P₆ is different in the constitution of a cover unit 105. In this cover unit 105, a water reservoir 105a having a protrusion 105b is made of the above-described synthetic resin. This water reservoir 105a is filled with water 106 and is covered and hermetically sealed, only at the bottom surface of the water reservoir 105a, with the above-described cover film 105c having a weak tear strength such as aluminum foil or aluminum deposited film. The cover unit 106 is formed by laminating it with a cover film 105d having adequate strength over the upper surface of an outer collar portion of the water reservoir 105a. As the cover film 105d, an aluminum deposited film or the like having greater tear strength than that of the cover film 105c and having a thickness of from about 30 to 120 µm is preferred.

This cosmetic patch package P₆ is obtained by stacking and laminating the cover unit 105 on the tray 101 having the net 102 and the cosmetic patch 103 stored in the storing recess 101a. The tray 101, net 102 and cosmetic patch 103 are similar to those described above so the description on them is omitted.

Such cosmetic patch package P₆ exhibits similar effects and advantages to those of the above-described cosmetic patch package P₁. In addition, since one cover film 105c must be broken by the protrusion 105b of the cover unit 105 at the time of use, only a small force is required to break the water reservoir 105 compared with the cosmetic patch package P₁ needing a force for breaking two cover films 105c, 104. In addition, since the cover film 105d of the cover unit 105 has great tear strength, there is no fear of the cover film 105 being broken when the cover unit 105 is peeled and the cosmetic patch 103 is taken out from the storing recess 101a. The cosmetic patch package according to this embodiment has such advantages in combination.

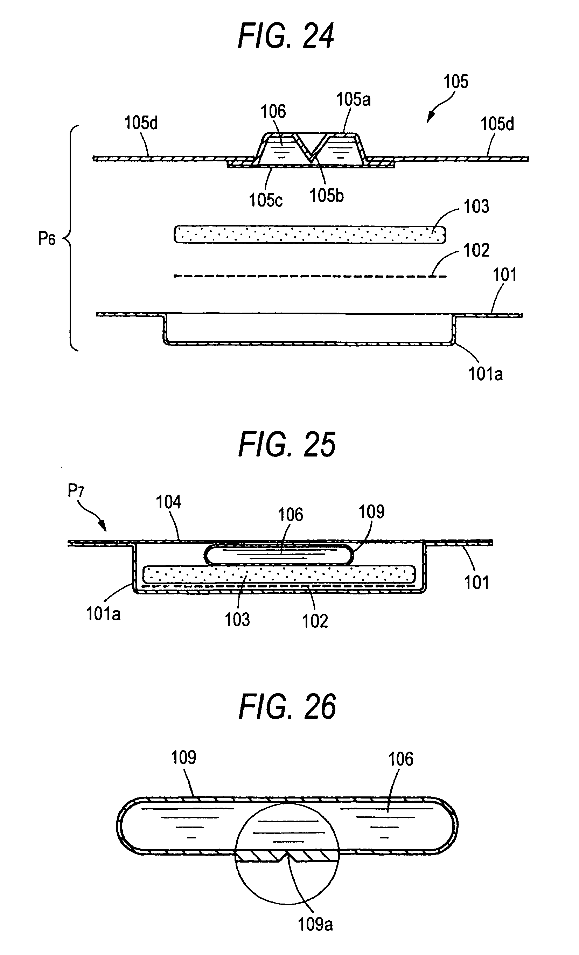

Fig. 24 is a cross-sectional view illustrating a cosmetic patch package according to a still further embodiment of the present invention; and Fig. 25 is a partially enlarged cross-sectional view illustrating a water reservoir bag of this cosmetic patch package.

The cosmetic patch package P₇ has the above-described net 102 laid over the bottom surface of the storing recess 101a of the tray 101. The cosmetic patch 103 having Vitamin C incorporated in the polyurethane thereof in a dry state and a water reservoir bag 109 are stacked over this net 102 and they are stored in the storing recess 101a. With the above-described cover film 104 laminated on the upper surface of the tray 101, the storing recess 101a is hermetically sealed.

The water reservoir bag 109 has water 106 filled in a bag made of a synthetic resin film such as polyethylene terephthalate, polyvinyl chloride, polypropylene or polyethylene. This bag has a notch 109a formed in a part thereof to facilitate breakage by pushing with a finger.

The cosmetic patch package P₇ having such a constitution is able to contain and retain vitamin C in the polyurethane gel of the cosmetic patch 103 stably without causing oxidation and hydrolysis of it. When an external force is applied to the water reservoir bag 109 from the cover film 104 by pushing downward with a finger at the time of use, the water reservoir bag 109 is broken easily and water is absorbed in the polyurethane gel of the cosmetic patch 103. When the cosmetic patch 103 is applied to an under-eye portion of the face, vitamin C transfers, together with water, to the skin and effects of vitamin C are exhibited.

A release amount of Vitamin C one hour after application to the skin is preferably from 3 to 30 wt%, more preferably from 15 to 25 wt% relative to the whole vitamin C content. Amounts greater than the above-described range are not preferred because they tend to cause inflammation or rash. Amounts below the above-described range, on the other hand, are not preferred from the viewpoints of whitening effects and release efficiency.

### EXAMPLES

The embodiments of the present invention will next be described more specifically by examples. It should however be borne in mind that these embodiments are not limited by these examples.

### Example 1

A tray as illustrated in Fig. 2 was molded using polyethylene terephthalate. A polyolefin film and a polyester nonwoven fabric having a basis weight of 40 g/m² were heat laminated and the resulting laminate was laid over the bottom of the tray with the nonwoven fabric up.

A polyol and a polyisocyanate each having a structure corresponding to the reference symbol of structural formula, molecular weight and alkylene oxide segments, and a catalyst, as described in Table 1, were mixed at a ratio as described in Table 1 and the mixture was added dropwise to fill the recess of the tray. By reacting at 60°C for a predetermined time, the gel layer of a polyurethane gel having the nonwoven fabric and polyolefin film stacked over the bottom surface and at the same time, having the nonwoven fabric embedded in the surface layer of the bottom surface was formed.

A water/ethanol solution of vitamin C was added dropwise to the upper surface of this gel layer to introduce therein vitamin C. Water and the solvent were removed from the polyurethane gel by drying under reduced pressure, whereby a cosmetic patch of the present invention containing 5 wt% of vitamin C in the gel layer of the polyurethane gel was obtained.

**Table 1**

| | | Parts |
|---|---|---|
| Polyol | Structural formula (D), R=H, molecular weight: 3000 | 100 |
| | (AO) = (CH₂CH₂O)ₘ(CH(CH₃)CH₂O)ₙ | |
| | random copolymer with m:n = 80:20 | |
| Polyisocyanate | Structural formula (G), I=1, molecular weight: 3000 | 35 |
| | (AO) = (CH₂CH₂O)ₘ(CH(CH₃)CH₂O)ₙ, R=(CH₂)₆ | |
| | random copolymer with m:n = 70:30 | |
| Catalyst | Dibutyltin dilaurate | 0.01 |

The storage stability and vitamin C release properties of the above-described cosmetic patch were analyzed using the following method.

After the cosmetic patch was put into an aluminum-deposited moisture-proof bag and stored at 40°C for 1 month, it was taken out from the bag. A change in the hue of the gel layer was visually observed, while a change in adhesion properties was studied by touching the gel layer with a finger. In addition, a change in vitamin C content was measured by high-performance liquid chromatography.

As a result, there was no change found in the hue and also in adhesion properties after the storage compared with those at the test start time. The remaining content of vitamin C including dehydroascorbic acid showing similar bioactivity to that of L-ascorbic acid was 96.5% of the vitamin C content at the test start time.

The cosmetic patch, together with a desiccant and an oxygen absorber, was put into the above-described moisture-proof bag and a change in hue, a change in adhesion properties, and a change in vitamin C content were studied in similar manners to the above-describe methods. There was no change found in hue and adhesion properties, while the remaining content of vitamin C was about 98% or greater of that of the test start time, showing improvement.

After dropwise addition of a small amount of water to the surface (surface to be applied to the skin) of the gel layer of the cosmetic patch, the cosmetic patch was applied to the face of each of 10 volunteers and the remaining content of vitamin C in the gel layer of the cosmetic patch peeled from the face after 1-hour application was measured. The vitamin C release properties of the cosmetic patch were studied by determining a release amount of vitamin C from a difference between the remaining vitamin C content and the vitamin C content of the cosmetic patch prior to use. The vitamin C content in the gel layer was determined by extracting vitamin C from the gel swelled with a solution hard to destroy vitamin C. As a result, the release rate of vitamin C after 1-hour application to the face of 10 volunteers was 21.6% on average.

### Example 2

As in Example 1, a polyolefin film and a nonwoven fabric were heat laminated and the resulting laminate was laid in the recess of a tray made of polyethylene terephthalate. A polyol and a polyisocyanate each having a structure corresponding to the reference symbol of structural formula, molecular weight and alkylene oxide segments, and a catalyst, as described in Table 2, were mixed at a ratio as described in Table 2 and the mixture was filled in the recess of the tray. By reacting at 60°C for a predetermined time, the gel layer of a polyurethane gel having the nonwoven fabric and polyolefin film stacked over the bottom surface and at the same time, having the nonwoven fabric embedded in the surface layer of the bottom surface was formed.

**Table 2**

| | | Parts |
|---|---|---|
| Polyol | Structural formula (D), R=H, molecular weight: 2000 | 100 |
| | (AO) = (CH₂CH₂O)ₘ(CH(CH₃)CH₂O)ₙ | |
| | random copolymer with m:n = 30:70 | |
| Polyisocyanate | Structural formula (G), I=1, molecular weight: 3000 | 50 |
| | (AO) = (CH₂CH₂O)ₘ(CH(CH₃)CH₂O)ₙ, R=(CH₂)₆ | |
| | random copolymer with m:n = 30:70 | |
| Catalyst | Dibutyltin dilaurate | 0.01 |

Vitamin E (tocopherol), a fat soluble vitamin, was dissolved in hexane and the resulting solution was added dropwise downward to the gel layer to impregnate it with the solution. The solvent was then evaporated, whereby a cosmetic patch containing 5 wt.% of vitamin E in the gel layer was obtained.

The resulting cosmetic patch, together with an oxygen absorber, was put into an aluminum-deposited moisture-proof outer bag and stored at 40°C for 1 month. As a result, there was no change found in the hue and adhesion properties and the Vitamin E content determined by reverse phase chromatography was 97.6% of that of the test start time.

A small amount of glycerin was applied to the surface of the gel layer in order to accelerate the release of vitamin E to the skin and a patch test was conducted. The release rate of vitamin E after application to the face of 10 volunteers for 1 hour was 14.7% on average.

### Example 3

As in Example 1, a polyolefin film and a nonwoven fabric were heat laminated and the resulting laminate was laid in the recess of a tray made of polyethylene terephthalate. A polyol and a polyisocyanate each having a structure corresponding to the reference symbol of structural formula, molecular weight and alkylene oxide segments, and a catalyst, as described in Table 3, were mixed at a ratio as described in Table 3 and the mixture was filled in the recess of the tray. By reacting at 60°C for a predetermined time, the gel layer of a polyurethane gel having the nonwoven fabric and polyolefin film stacked over the bottom surface and at the same time, having the nonwoven fabric embedded in the surface layer of the bottom surface was formed.

**Table 3**

| | | Parts |
|---|---|---|
| Polyol | Structural formula (D), R=H, molecular weight: 1700 | 100 |
| | (AO) = (CH₂CH₂O)ₘ(CH(CH₃)CH₂O)ₙ | |
| | random copolymer with m:n = 70:30 | |
| Polyisocyanate | Structural formula (G), I=1, molecular weight: 2600 | 50 |
| | (AO) = (CH(CH₃)CH₂O)ₙ(CH(CH₃)CH₂O)ₙ, R=(CH₂)₆ | |
| | random copolymer with m:n = 50:50 | |
| Catalyst | Dibutyltin dilaurate | 0.01 |

A water/ethanol solution of vitamin C as employed in Example 1 was added dropwise to the upper surface of the gel layer to impregnate it with the solution. Water and the solvent were then removed by drying under reduced pressure, whereby a cosmetic patch containing 5 wt% of vitamin C in the gel layer of the polyurethane gel was obtained.

The resulting cosmetic patch, together with an oxygen absorber, was put into an aluminum-deposited moisture-proof outer bag and stored at 40°C for 1 month. As a result, there was no change found in the hue and adhesion properties and the vitamin C content determined by high performance liquid chromatography was 98.1% of that of the test start time.

Vitamin C was extracted from the patch after 1-hour application and its amount was determined by high performance liquid chromatography. As a result, it was found that a release rate of vitamin C to the skin was 16.9%.

### Example 4

A preparation method of a patch having, introduced therein, vitamin C which is a typical example of unstable water soluble vitamins and niacinamide which is a typical example of stable water soluble vitamins will next be described. In a similar method and with a similar composition to those of Example 1, the gel layer of a polyurethane gel was formed. A water/ethanol solution of Vitamin C was added dropwise to the upper surface of the gel layer in an amount half of the amount of Example 1, followed by the dropwise addition of a water/ethanol solution of niacinamide in an amount half of the amount of Example 1. The solvent was then evaporated, whereby a cosmetic patch containing vitamin C and niacinamide, each in an amount of 2.5 wt%, was obtained.

The cosmetic patch, together with a desiccant and an oxygen absorber, was put in an aluminum-deposited, moisture-proof outer bag and stored at 40°C for 1 month. As a result, no change was found in both hue and adhesion properties. The vitamin C content and niacinamide content determined by high performance liquid chromatography were 96.2% and 100.8% of those at the test start time, respectively.

After 1-hour application, vitamin C and niacinamide were extracted from the cosmetic patch and their amounts were determined by high performance liquid chromatography. As a result, it was found that the release rates of vitamin C and niacinamide to the skin were 15.2% and 22.4%, respectively.

### Example 5

As in Example 1, a polyolefin film and a nonwoven fabric were heat laminated and the resulting laminate was laid in the recess of a tray made of polyethylene terephthalate. A polyol and a polyisocyanate each having a structure corresponding to the reference symbol of structural formula, molecular weight and alkylene oxide segments, and a catalyst, as described in Table 4, were mixed at a ratio as described in Table 4 and the mixture was added dropwise to the recess of the tray to fill it with the mixture. By reacting at 60°C for a predetermined time, the gel layer of a polyurethane gel having the nonwoven fabric embedded in the surface layer of the bottom surface and having a resin film stacked over the bottom surface was formed. A water/ethanol solution of vitamin C was added dropwise to the upper surface of the gel layer to impregnate it with the solution. Water and solvent were removed by drying under reduced pressure, whereby a cosmetic patch containing 5 wt% of vitamin C was obtained.

The resulting cosmetic patch, together with an oxygen absorber, was put into an aluminum-deposited moisture-proof outer bag and stored at 40°C for 1 month. As a result, no change was found in both the hue and adhesion properties and the vitamin C content determined by high performance liquid chromatography was 97.0% of that of the test start time.

Vitamin C was extracted from the patch after application for 1 hour and its amount was determined by high performance liquid chromatography. As a result, it was found that a release rate of vitamin C to the skin was 6.6%.

**Table 4**

| | | Parts |
|---|---|---|
| Polyol | Structural formula (B), I=1, molecular weight: 1000 | 100 |
| | (AO) = (CH₂CH₂O)ₘ | |
| | Structural formula (D), R=H, molecular weight: 3000 | 20 |
| | (AO) = (CH₂CH₂O)ₘ(CH(CH₃)CH₂O)ₙ | |
| | random copolymer with m:n = 80:20 | |
| Polyisocyanate | Structural formula (G), 1=1, molecular weight: 1600 | 60 |
| | (AO) = (CH₂CH₂O)ₘ, R=(CH₂)₆ | |
| Catalyst | Dibutyltin dilaurate | 0.01 |

### Example 6

In a similar method and with a similar composition to those of Example 2, the gel layer of a polyurethane gel was formed. A water/ethanol solution of vitamin C was added dropwise to the upper surface of the gel layer in an amount equal to that of Example 1. A portion of vitamin C remained on the surface of the gel layer without being absorbed therein. The dropwise addition amount of the vitamin C solution was adjusted so as to avoid remaining of vitamin C on the surface of the gel layer. As a result, after drying under reduced pressure, a cosmetic patch containing 2 wt% of vitamin C was obtained.

The resulting cosmetic patch, together with an oxygen absorber, was put into an aluminum-deposited moisture-proof outer bag and stored at 40°C for 1 month. As a result, no change was found in both the hue and adhesion properties and the vitamin C content determined by high performance liquid chromatography was 97.3% of that of the test start time.

Vitamin C was extracted from the patch after application for 1 hour and its amount was determined by high performance liquid chromatography. As a result, it was found that a release rate of vitamin C to the skin was 24.3%.

### Comparative Example 1

A poly(acrylic acid) hydrogel adhesive layer having a composition as shown in Table 5 was formed over a similar film/nonwoven fabric laminate to that employed in Example 1. A water/ethanol solution of vitamin C was added dropwise to the layer to impregnate it with the solution. Water and the solvent were removed by drying under reduced pressure, whereby a very brittle patch was obtained in the curled form. Although water was added dropwise to the patch in order to provide it for use, curling prevented water from spreading all over the adhesive layer.

Since drying causes the above-described problem upon application, a storage stability test was performed on the assumption of the storage and application of the patch in a hydrous state. After introduction of vitamin C into the adhesive layer, the patch was stored in the presence of an oxygen absorber at 40°C without drying. One month later, the patch turned a pale brown and the Vitamin C content decreased to 12.4% of that of the test start time.

**Table 5**

| | Parts |
|---|---|
| Polyacrylic acid | 2.00 |
| Sodium alginate | 2.00 |
| Hydroxyethyl cellulose | 2.00 |
| Kaolin | 3.00 |
| Synthetic hydrotalcite | 0.10 |
| Aluminum hydroxide | 0.05 |
| Sorbitol | 25.00 |
| PEG400 | 10.00 |
| Purified water | 55.75 |

Tests for determining the storage stability and Vitamin C release properties of the cosmetic patch package of the present invention will next be described.

### Example 7

### (Preparation of cosmetic patch package)

A polyethylene terephthalate film was used as a mold release film and on this film, a frame was placed. A nonwoven fabric piece cut out from a polyester nonwoven fabric having a basis weight of 37 g/m² into a shape substantially similar to that of the frame was placed inside of the frame. A polyol and a polyisocyanate each having a structure corresponding to the reference symbol of structural formula, molecular weight and alkylene oxide segments, and a catalyst, as described in Table 6, were mixed at a ratio as described in Table 6 and the resulting mixture was cast onto the nonwoven fabric piece inside the frame, followed by reaction at heating temperature of 65°C for 24 hours, whereby a polyurethane gel (segmented polyurethane gel) having a nonwoven fabric piece stacked over the surface layer of the bottom surface of the gel, a thickness of 1.2 mm and a volume of 1.2 cm³ was formed.

A nylon net (diameter of monofilament: 0.2 mm, mesh size: 50 mesh) was stacked over the segmented polyurethane gel. A 14% water/ethanol solution of vitamin C (Vitamin C:water:ethanol weight ratio = 1:3:3) was added dropwise to cause the polyurethane gel to contain vitamin C. The frame was then removed, and water and ethanol were removed by drying under reduced pressure, whereby a cosmetic patch having, incorporated in the polyurethane gel thereof, 5 wt% of vitamin C was obtained.

A tray molded from polyethylene terephthalate and having a storing recess was placed over the cosmetic patch and the nylon net. After the cosmetic patch and nylon net were stored in the storing recess by turning the tray upside down, the tray was laminated with an aluminum foil to hermetically seal the storing recess. A cover unit obtained by pouring 1.0 ml of water in a water reservoir and hermetically sealing with an aluminum foil was stacked over the tray, whereby a cosmetic patch package of the present invention was prepared.

**Table 6**

| | | Parts |
|---|---|---|
| Polyol | Structural formula (B), I=1, molecular weight: 7000 | 80 |
| | (AO) = (CH₂CH₂O)ₘ(CH(CH₃)CH₂O)ₙ | |
| | random copolymer with m:n = 80:20 | |
| | Structural formula (D), R=H, molecular weight: 3000 | 100 |
| | (AO) = (CH₂CH₂O)ₘ(CH(CH₃)CH₂O)ₙ | |
| | random copolymer with m:n = 80:20 | |
| Polyisocyanate | Structural formula (G), I=1, molecular weight: 2900 | 45 |
| | (AO) = (CH₂CH₂O)ₘ(CH(CH₃)CH₂O)ₙ, R=(CH₂)₆ | |
| | random copolymer with m:n = 70:30 | |
| Catalyst | Dibutyltin dilaurate | 0.01 |

### (Storage stability)

After the cosmetic patch package obtained above was stored at 40°C for 1 month, the cosmetic patch was taken out from the tray. A change in the hue of the polyurethane gel was visually observed, while a change in adhesion properties was studied by touching the gel with a finger. A change in the vitamin C content was determined in accordance with the indophenol xylene colorimetry as described in the Japanese Pharmacopoeia Guideline (colorimetry at 500 nm by adding an excessive indophenol reagent to a metaphosphoric acid solution of ascorbic acid and then dissolving, in xylene, a colorant remaining after reductive bleaching).

As a result, there was no change found in the hue and also in adhesion properties after the storage compared with those at the test start time. The remaining content of vitamin C including dehydroascorbic acid showing similar bioactivity to L-ascorbic acid was 96.5% of the vitamin C content at the test start time.

The cosmetic patch, together with a desiccant and an oxygen absorber, was stored in the storing recess of the tray and the tray was then hermetically sealed. A change in hue, a change in adhesion properties, and a change in Vitamin C content were studied in similar manners. As a result, there was no change found in hue and adhesion properties, while the remaining content of Vitamin C was about 98% or greater of that of the test start time, showing improvement.

### (Vitamin C release properties)

The water reservoir of the cover which had been laid over the tray was broken by pushing with a finger and water running into the storing recess of the tray was allowed to stand until it penetrated and was absorbed in the polyurethane gel of the cosmetic patch. The resulting patch was applied to the face of each of 10 volunteers for 1 hour. By determining a release rate of vitamin C from a difference between a vitamin C content remaining in the polyurethane gel of the cosmetic patch peeled from the face and a vitamin C content of another cosmetic patch prior to use, the vitamin C release properties of the cosmetic patch were studied. The vitamin C in the polyurethane gel was determined by cutting the polyurethane gel of the cosmetic patch into small pieces, and extracting vitamin C from these pieces swelled for analysis with a solution having a composition preventing decomposition of vitamin C. As a result, a release rate of vitamin C after application to the face of 10 volunteers for 1 hour was 21.6% on average.

### Example 8

### (Preparation of cosmetic patch package)

In a similar manner to that employed in Example 7 except that a mixture obtained by mixing a polyol and a polyisocyanate each having a structure corresponding to the reference symbol of structural formula, molecular weight and alkylene oxide segments, and a catalyst, as described in Table 7 at a mixing ratio as described in Table 7 was used and heating temperature of it was changed to 60°C, a cosmetic patch package was prepared.

**Table 7**

| | | Parts |
|---|---|---|
| Polyol | Structural formula (B), I=1, molecular weight: 3000 | 200 |
| | (AO) = (CH₂CH₂O)ₘ | |
| | Structural formula (D), R=H, molecular weight: 600 | 100 |
| | (AO) = (CH₂CH₂O)ₘ | |
| Polyisocyanate | Structural formula (G), I=1, molecular weight: 2000 | 100 |
| | (AO) = (CH(CH₃)CH₂O)ₙ, R=(CH₂)₆ | |
| Catalyst | Dibutyltin dilaurate | 0.01 |

### (Storage stability)

In a similar manner to Example 7, storage stability of the cosmetic patch package obtained above was studied. As a result, there was no change found in the hue and also in adhesion properties after the storage compared with those at the test start time. The remaining content of Vitamin C including dehydroascorbic acid showing similar bioactivity to L-ascorbic acid was 96% of the vitamin C content at the test start time.

### (Vitamin C release properties)

The water reservoir of the cover which had been laid over the tray was broken by pushing it with a finger and the cosmetic patch which absorbed water therein was applied to the face of 10 volunteers for 1 hour. In a similar manner to that employed in Example 7, a release rate of vitamin C was determined. As a result, the release rate of vitamin C was 11% on average.

### Comparative Example 2

For comparison, a polyacrylic acid gel was prepared using a commercially available raw material for polyacrylic acid gel. A 14% water/ethanol solution of vitamin C similar to that employed in Example 7 was added dropwise to the gel to impregnate it with the solution. In several days, the polyacrylic acid gel turned yellow with the passage of time and it was not suited for the preparation of a cosmetic patch.

The present invention was described in detail by specific embodiments. It is apparent for those skilled in the art that it can be changed or deformed to an extent not departing from the intention and scope of the present invention.

The present application is based on Japanese Patent Application (Japanese Patent Application No. 2003-158250) filed on June 3, 2003, Japanese Patent Application (Japanese Patent Application No. 2003-337330) filed on September 29, 2003 and Japanese Patent Application (Japanese Patent Application No. 2003-337331) filed on September 29, 2003. They are all incorporated herein by reference.

### INDUSTRIAL APPLICABILITY

The present invention makes it possible to provide a cosmetic patch which can contain therein either a water soluble vitamin or a fat soluble vitamin, particularly when the vitamin is vitamin C, can contain and retain vitamin C stably while avoiding its deterioration due to oxidation and hydrolysis and deterioration of the gel, and is equipped with a desired level of adhesive force; and a using method of a cosmetic patch capable of effectively transferring and impregnating vitamin C to the skin.

The present invention also makes it possible to provide a cosmetic patch package capable of preventing deterioration due to oxidation and hydrolysis of a water soluble vitamin (particularly, vitamin C) and also deterioration of a gel by an acidic substance which will otherwise be generated by the oxidation and hydrolysis, capable of effectively releasing the water soluble vitamin at the time of use, and being equipped with a desired level of adhesive force.

## Claims

1. A cosmetic patch which comprises an adhesive polyurethane gel which has hydrophilic alkylene oxide segments and hydrophobic alkylene oxide segments and has most or all of said segments in liquid form at normal temperature, and a vitamin contained therein.

2. A cosmetic patch according to claim 1, wherein the hydrophilic alkylene oxide segments are ethylene oxide chains and the hydrophobic alkylene oxide segments are propylene oxide chains.

3. A cosmetic patch according to claim 2, wherein the polyurethane gel has ethylene oxide chains and propylene oxide chains at a molar ratio of from 50 to 90 : from 50 to 10 and the vitamin is a water soluble vitamin.

4. A cosmetic patch according to claim 2, wherein the polyurethane gel has ethylene oxide chains and propylene oxide chains at a molar ratio of from 50 to 10 : from 50 to 90 and the vitamin is a fat soluble vitamin.

5. A cosmetic patch according to claim 1, wherein a vitamin is incorporated in an adhesive polyurethane gel having segments made of a copolymer of ethylene oxide and propylene oxide and having most or all of said segments in liquid form at normal temperature.

6. A cosmetic patch according to claim 1 or 5, wherein the polyurethane gel forms a gel layer.

7. A cosmetic patch according to any one of claims 1, 3, 5 and 6, wherein the vitamin is vitamin C and the polyurethane gel is free of a solvent for vitamin C.

8. A cosmetic patch according to claim 6, wherein the gel layer has, on one surface thereof, a nonwoven fabric and a resin film stacked one after another.

9. A using method of a cosmetic patch, which comprises, just before use of a cosmetic patch obtained by incorporating a water soluble vitamin in a gel layer, which is made of an adhesive polyurethane gel comprising ethylene oxide segments and propylene oxide segments, or segments composed of a copolymer of ethylene oxide and propylene oxide and having most or all of these segments in liquid form at normal temperature, in a dry state in the absence of a solvent for the water soluble vitamin; impregnating the gel layer of the cosmetic patch with water to dissolve the water soluble vitamin therein; and applying the cosmetic patch to skin to transfer the water soluble vitamin and water thereinto.

10. A using method according to claim 9, wherein water is added to the cosmetic patch, which has been stored in a concave body while having a net made of a synthetic resin laid at the bottom surface of the patch, to impregnate the patch with water and after removal of the net, the cosmetic patch is applied to the skin.

11. A cosmetic patch package which comprises:
a cosmetic patch which comprises a substantially water-free adhesive polyurethane gel which has hydrophilic alkylene oxide segments and hydrophobic alkylene oxide segments and has most or all of said segments in liquid form at normal temperature, and a water soluble vitamin contained therein;
a tray having formed therein a storing recess for storing the cosmetic patch, and
a cover unit which is to be laid over the tray, has a water reservoir containing water to be fed to the storing recess, and opens the bottom surface of the reservoir by the application of an external force to the reservoir.

12. A cosmetic patch package which comprises:
a cosmetic patch which comprises a substantially water-free adhesive polyurethane gel which has hydrophilic alkylene oxide segments and hydrophobic alkylene oxide segments and has most or all of said segments in liquid form at normal temperature, and a water soluble vitamin contained therein;
a tray having formed therein a storing recess for storing the cosmetic patch, and
a water reservoir bag which is stored in the storing recess of the tray after laid over the cosmetic patch, and opens by the application of an external force.

13. A cosmetic patch package according to claim 11 or 12, wherein the water soluble vitamin is vitamin C.

14. A cosmetic patch package according to claim 11 or 12, wherein the polyurethane gel has hydrophilic ethylene oxide segments and hydrophobic propylene oxide segments, or has segments composed of a copolymer of hydrophilic ethylene oxide and hydrophobic propylene oxide.

15. A cosmetic patch package according to claim 11, wherein the water reservoir of the cover unit laid over the tray is located above the storing recess.

16. A cosmetic patch package according to claim 11, wherein the storing recess is formed at two sites of the tray, a connecting channel for connecting said two storing recesses is formed in the tray, and the cover unit is laid over the tray so that the water reservoir is located above the connecting channel.

17. A cosmetic patch package according to any one of claims 11 to 16, wherein the storing recess of the tray has small concavity and convexity formed on the bottom surface thereof.

18. A cosmetic patch package according to any one of claims 11 to 16, wherein a net is laid over the bottom surface of the storing recess of the tray.

19. A cosmetic patch package according to claim 11, wherein an opening leading to the exterior is formed between the tray and the cover unit to be laid thereover.

20. A cosmetic patch package according to claim 16, wherein one of the tray and cover is equipped with a protrusion and the other one is equipped with a recess in which the protrusion is fit so that the water reservoir of the cover unit is located above the connecting channel of the tray.

21. A cosmetic patch package according to claim 11, wherein the tray and the cover unit are formed so that the periphery of one of the tray and cover protrudes from that of the other one when the tray and the cover unit are overlapped each other.
